# EUROPEAN PATENT APPLICATION

(11) **EP 2 325 193 A2**
(43) Date of publication of application: **25.05.2011**
(21) Application number: 10012442.9
(22) Date of filing: 04.11.2002
(51) Int. Cl.: C07H 21/02, C07H 21/04, A61K 48/00

(54) **Methods and compositions for therapeutic use of RNA interference**

(30) Priority: 02.11.2001 US 336314 P; 05.11.2001 US 337304 P; 15.10.2002 US 418909 P
(62) Divisional of application: 02807994.5
(71) Applicant: Insert Therapeutics, Inc., Pasadena, CA 91107 (US)
(72) Inventor: Davis, Mark E., Pasadena, CA 91105 (US); Pun, Suzie Hwang, Seattle, WA 98101 (US); Jensen, Gregory, S., Pasadena, CA 91101 (US)
(74) Representative: Graf von Stosch, Andreas

(57) **Abstract**

The present invention provides methods and compositions for attenuating expression of a target gene in vivo. In general, the method includes administering RNAi constructs (such as small-interfering RNAs (i.e., siRNAs) that are targeted to particular mRNA sequences, or nucleic acid material that can produce siRNAs in a cell), in an amount sufficient to attenuate expression of a target gene by an RNA interference mechanism, e.g., in a sequence-dependent, PKR-independent manner. In particular, the subject method can be used to alter the growth, survival or differentiation of cells for therapeutic and cosmetic purposes

## Description

### Background of the Invention

The structure and biological behavior of a cell is determined by the pattern of gene expression within that cell at a given time. Perturbations of gene expression have long been acknowledged to account for a vast number of diseases including, numerous forms of cancer, vascular diseases, neuronal and endocrine diseases. Abnormal expression patterns, in form of amplification, deletion, gene rearrangements, and loss or gain of function mutations, are now known to lead to aberrant behavior of a disease cell. Aberrant gene expression has also been noted as a defense mechanism of certain organisms to ward off the threat of pathogens.

One of the major challenges of medicine has been to regulate the expression of targeted genes that are implicated in a wide diversity of physiological responses. While over-expression of an exogenously introduced transgene in a eukaryotic cell is relatively straightforward, targeted inhibition of specific genes has been more difficult to achieve. Traditional approaches for suppressing gene expression, including site-directed gene disruption, antisense RNA or co-suppress or injection, require complex genetic manipulations or heavy dosages of suppressors that often exceeds the toxicity tolerance level of the host cell.

RNA interference (RNAi) is a phenomenon describing double-stranded (ds)RNA-dependent gene specific posttranscriptional silencing. Initial attempts to harness this phenomenon for experimental manipulation of mammalian cells were foiled by a robust and nonspecific antiviral defense mechanism activated in response to long dsRNA molecules. Gil et al. Apoptosis 2000, 5:107-114. The field was significantly advanced upon the demonstration that synthetic duplexes of 21 nucleotide RNAs could mediate gene specific RNAi in mammalian cells, without invoking generic antiviral defense mechanisms. Elbashir et al. Nature 2001, 411:494-498; Caplen et al. Proc Natl Acad Sci 2001, 98:9742-9747. As a result, small-interfering RNAs (siRNAs) have become powerful tools to dissect gene function. The chemical synthesis of small RNAs is one avenue that has produced promising results. Numerous groups have also sought the development of DNA-based vectors capable of generating such siRNA within cells. Several groups have recently attained this goal and published similar strategies that, in general, involve transcription of short hairpin (sh)RNAs that are efficiently processed to form siRNAs within cells. Paddison et al. PNAS 2002, 99:1443-1448; Paddison et al. Genes & Dev 2002, 16:948-958; Sui et al. PNAS 2002, 8:5515-5520; and Brummelkamp et al. Science 2002, 296:550-553. These reports describe methods to generate siRNAs capable of specifically targeting numerous endogenously and exogenously expressed genes.

### Summary of the Invention

One aspect of the present invention provides a stable respiratory formulation comprising RNAi constructs formulated for pulmonary or nasal delivery of a therapeutically effective amount of said RNAi constructs to the lungs of a patient. In certain embodiments, the RNAi constructs are formulated as microparticles having an average diameter less than 20 microns, and more preferably, having an average diameter of 0.5 to 10 microns. In certain embodiments, the microparticles are formed from biodegradable polymers. In certain embodiments, the microparticles are formed from one or more polymers selected from the group consisting of polysaccharides, diketopiperazines, poly(hydroxy acids), polyanhydrides, polyesters, polyamides, polycarbonates, polyalkylenes, poly vinyl compounds, polysiloxanes, polymers of acrylic and methacrylic acids, polyurethanes, celluloses, poly(butic acid), poly(valeric acid), and poly(lactide-co-caprolactone), or co-polymers thereof.

In certain embodiments, the microparticles are formed by solvent evaporation, spray drying, solvent extraction or hot melt encapsulation; while in other embodiments, the microparticles are in dry or lyophilized form. In other embodiments, the RNAi constructs are formulated in liposomes.

In still other embodiments, the RNAi constructs, are formulated as supramolecular complexes including a multi-dimensional polymer network. Preferably, the supramolecular complexes are formed from cationic polymers such as poly(L)lysine (PLL), polyethylenimine (PEI), β-cyclodextrin containing polymers (βCD-polymers) or co-polymers thereof. More preferably, the supramolecular complexes are formed from cyclodextrin-modified polymers, for example, the cyclodextrin-modified poly(ethylenimine) having a structure of the formula: 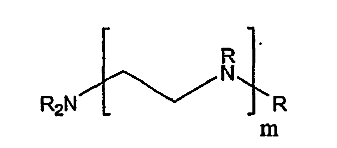 wherein
R represents, independently for each occurrence, H, lower alkyl, a 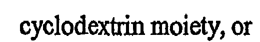 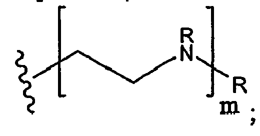 and
m, independently for each occurrence, represents an integer from 2-10,000, preferably from 10 to 5,000, or from 100 to 1,000.

In certain embodiments, the respiratory formulation comprising RNAi constructs include a propellant.

In certain embodiments, the respiratory formulation is contained in a metered dose inhaler, a dry powder inhaler or an air-jet nebulizer. In a preferred embodiment, the RNAi construct is formulated in an amount to provide a therapeutically effective amount in one to ten meter doses.

Another aspect of the invention provides a metered dose aerosol dispenser containing an aerosol pharmaceutical composition for pulmonary or nasal delivery comprising a respirable formulation of RNAi constructs.

Yet another aspect of the invention provides a method for affecting systemic administration of an RNAi construct comprising administering to a patient, by way of pulmonary administration, a respirable formulation of RNAi constructs which is taken up in an amount in the deep lung to deliver a systemic dose of said RNAi construct.

Still another aspect of the invention provides a pharmaceutical preparation comprising at least one RNAi construct formulated for pulmonary or nasal delivery, and a pharmaceutically acceptable carrier. Optionally, the pharmaceutically acceptable carrier is selected from pharmaceutically acceptable salts, ester, and salts of such esters. In certain preferred embodiments, the present invention provides a pharmaceutical package comprising the pharmaceutical preparation which includes at least one RNAi construct formulated for pulmonary or nasal delivery and a pharmaceutically acceptable carrier, in association with instructions (written and/or pictorial) for administering the preparation to a human patient.

Another aspect of the present invention provides a composition comprising one or more RNAi constructs formulated in a supramolecular complex and in an amount sufficient to attenuate expression of a target gene in treated cells through an RNA interference mechanism. For example, the RNAi construct is an small-interfering RNA (siRNA), preferably being 19-30 base pairs in length. Alternatively, the RNAi construct is an expression vector having a coding sequence that is transcribed to produce one or more transcriptional products that produce siRNA in the treated cells. Optionally, the RNAi construct is a hairpin RNA which is processed to an siRNA in said treated cells. In certain embodiments, the composition is administered for treatment of cells *in vivo* or *in vitro.*

In certain embodiments, the supramolecular complexes are aggregated into particles having an average diameter of between 20 and 500 nm, and more preferably, between 20 and 200 nm.

To further illustrate, the supramolecular complex can be a multi-dimensional polymer network including linear polymers or branched polymers. Exemplary polymers are cationic polymers such as poly(L)Iysine (PLL), polyethylenimine (PEI), β-cyclodextrin containing polymers (βCD-polymers) or co-polymers thereof. In certain embodiments, the supramolecular complexes are formed from cyclodextrin-modified polymers, for example, the cyclodextrin-modified poly(ethylenimine) having a structure of the formula: 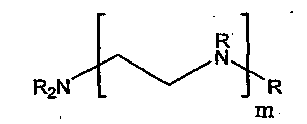 wherein
R represents, independently for each occurrence, H, lower alkyl, a cyclodextrin moiety, or 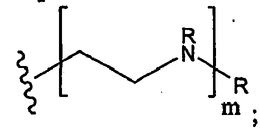 and
m, independently for each occurrence, represent an integer from 2-10,000, preferably from 10 to 5,000, or from 100 to 1,000.

Another aspect of the present invention provides a method for attenuating expression of a target gene of a cell *in vivo,* comprising administering an RNAi construct, formulated in a supramolecular complex, in an amount sufficient to attenuate expression of the target gene through an RNA interference mechanism, and thereby alter the growth, survival or differentiation of treated cells.

Yet another aspect of the invention provides a pharmaceutical preparation comprising at least one RNAi construct formulated in a supramolecular complex, and a pharmaceutically acceptable carrier. Optionally, the pharmaceutically acceptable carrier is selected from pharmaceutically acceptable salts, ester, and salts of such esters. In certain preferred embodiments, the present invention provides a pharmaceutical package comprising the pharmaceutical preparation which includes at least one RNAi construct formulated in a supramolecular complex and a pharmaceutically acceptable carrier, in association with instructions (written and/or pictorial) for administering the preparation to a human patient.

Another aspect of the present invention provides a coating for use on a surface of a medical device, comprising a polymer matrix having RNAi constructs dispersed therein, which RNAi constructs are eluted from the matrix when implanted at site in a patient's body and alter the growth, survival or differentiation of cells in the vicinity of the implanted device. Exemplary medical devices include screws, plates, washers, sutures, prosthesis anchors, tacks, staples, electrical leads, valves, membranes, catheters, implantable vascular access ports, blood storage bags, blood tubings, central venous catheters, arterial catheters, vascular grafts, intraaortic balloon pumps, heart valves, cardiovascular sutures, artificial hearts, pacemakers, ventricular assist pumps, extracorporeal devices, blood filters, hemodialysis units, hemoperfasion units, plasmapheresis units, and filters adapted for deployment in a blood vessel. A certain preferred embodiment provides a coated stent.

In certain embodiments, the RNAi construct of the coating is an small-interfering RNA (siRNA), preferably being 19-30 base pairs in length. Alternatively, the RNAi construct is an expression vector having a coding sequence that is transcribed to produce one or more transcriptional products that produce siRNA in the treated cells. Optionally, the RNAi construct is a hairpin RNA which is processed to an siRNA in the treated cells.

To illustrate, the RNAi construct of the coating can be one that attenuates at least one target gene selected from cyclin dependent kinases, c-myb, c-myc, proliferating cell nuclear antigen (PCNA), transforming growth factor-beta (TGF-beta), and transcription factors nuclear factor kappaB (NF-κB), E2F, HER-2/neu, PKA, TGF-alpha, EGFR, TGF-beta, IGFIR, P12, MDM2, BRCA, Bcl-2, VEGF, MDR, ferritin, transferrin receptor, IRE, C-fos, HSP27, C-raf, and metallothionein genes.

In certain preferred embodiments, the RNAi construct inhibits expression of a gene so as to attenuate proliferation and/or migration of smooth muscle cells.

Still another aspect of the present invention provides a method for coating a medical device with one or more RNAi constructs, comprising:
a) formulating the RNAi construct for coating a surface of a device such that said RNAi constructs are eluted from the surface when the device is implanted at site in a patient's body; and
b) coating the formulated RNAi construct on a medical device,
wherein the medical device coated with the RNAi construct attenuates expression of one or more genes in cells in the vicinity of the implanted device.

Another aspect of the present invention provides a composition comprising one or more RNAi constructs formulated for percutaneous intrapericardial delivery to an animal. In one embodiment, the RNAi construct of the composition attenuates expression of a gene resulting in increased angiogenesis and/or reduced ischemic damage in and around a myocardial infarct. Optionally, the RNAi construct is systemically available and attenuates expression of one or more genes in cells distal to the pericardial space.

In certain embodiments, the RNAi construct of the composition is encapsulated or associated with liposonies. For example, the liposomes are cationic liposomes formed from cationic vesicle-forming lipids. Optionally, the liposomes of the composition have an average diameter of less than about 200 nm.

In other embodiments, the RNAi construct is formulated as supramolecular complexes including a multi-dimensional polymer network. Preferably, the polymers are cationic polymers such as poly(L)lysine (PLL), polyethylenimine (PEI), β-cyclodextrin containing polymers (βCD-polymers) or co-polymers thereof.

In certain embodiments, the supramolecular complexes of the composition are formed from cyclodextrin-modified polymers, for example, the cyclodextrin-modified poly(ethylenimine) having a structure of the formula: 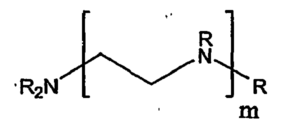 wherein
R represents, independently for each occurrence, H, lower alkyl, a cyclodextrin moiety, or 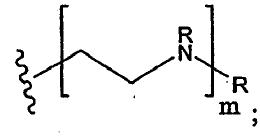 and
m, independently for each occurrence, represents an integer from 2-10,000, preferably from 10 to 5,000, or from 100 to 1,000.

In certain embodiments; the RNAi construct of the composition is an small-interfering RNA (siRNA), preferably being 19-30 base pairs in length. Alternatively, the RNAi construct is an expression vector having a coding sequence that is transcribed to produce one or more transcriptional products that produce siRNA in the treated cells. Optionally, the RNAi construct is a hairpin RNA which is processed to an siRNA in the treated cells. In a preferred embodiment, the animal of the composition is a human.

In one embodiment, the present invention provides a pharmaceutical preparation comprising at least one RNAi construct formulated for percutaneous intrapericardial delivery, and a pharmaceutically acceptable carrier. Optionally, the pharmaceutically acceptable carrier is selected from pharmaceutically acceptable salts, ester, and salts of such esters. In certain preferred embodiments, the present invention provides a pharmaceutical package comprising the pharmaceutical preparation which includes at least one RNAi formulated for percutaneous intrapericardial delivery and a pharmaceutically acceptable carrier, in association with instructions (written and/or pictorial) for administering the preparation to a human patient.

Still another aspect of the present invention provides a method for percutaneous intrapericardial delivery of one or more RNAi constructs *in vivo,* comprising administering a formulation of RNAi constructs to the pericardial space of an animal, wherein the RNAi constructs are present in an amount sufficient to attenuate expression of one or more target genes of cells of the treated animal. For example, the pericardial space is used as a delivery reservoir for the RNAi constructs. In certain embodiments, the RNAi construct of the method is delivered locally to the heart and surrounding vasculature. In other embodiments, the RNAi construct of the method is used for reducing proliferation and/or migration of smooth muscle cells, and more preferably, for treating myocardial infarction.

Another aspect of the present invention provides a composition comprising one or more RNAi constructs formulated in liposomes for attenuating expression of a target gene of cells in vivo through an RNA interference mechanism. In certain preferred embodiments, the RNAi construct is an small-interfering RNA (siRNA), preferably being 19-30 base pairs in length. Alternatively, the RNAi construct is an expression vector having a coding sequence that is transcribed to produce one or more transcriptional products that produce siRNA in the treated cells. Optionally, the RNAi construct is a hairpin RNA which is processed to an siRNA in the treated cells. Preferably, the cell is a mammalian cell, such as a human cell.

In certain embodiments, the liposomes of the composition are cationic liposomes including cationic vesicle-forming lipids. In other embodiments, the liposomes have an average diameter of less than about 200 nm.

Still another aspect of the present invention provides a method for attenuating expression of a target gene of cells of a patient, comprising administering RNAi constructs formulated in liposomes and in an amount sufficient to attenuate expression of a target gene through an RNA interference mechanism, so as to thereby alter the growth, survival or differentiation of said cells. In certain preferred embodiments, the RNAi construct of the method is an small-interfering RNA (siRNA), preferably being 19-30 base pairs in length. Alternatively, the RNAi construct is an expression vector having a coding sequence that is transcribed to produce one or more transcriptional products that produce siRNA in the treated cells. Optionally, the RNAi construct is a hairpin RNA which is processed to an siRNA in the treated cells. Preferably, the cell the method is a mammalian cell, such as a human cell.

In certain embodiments, the liposomes of the method are cationic liposomes including cationic vesicle-forming lipids. In other embodiments, the liposomes have an average diameter of less than about 200 nm.

In certain embodiments, the present invention provides a pharmaceutical preparation comprising at least one RNAi construct formulated in liposomes, and a pharmaceutically acceptable carrier. Optionally, the pharmaceutically acceptable carrier is selected from pharmaceutically acceptable salts, ester, and salts of such esters. In certain preferred embodiments, the present invention provides a pharmaceutical package comprising the pharmaceutical preparation which includes at least one RNAi formulated in liposomes and a pharmaceutically acceptable carrier, in association with instructions (written and/or pictorial) for administering the preparation to a human patient.

Another aspect of the present invention provides a composition comprising one or more RNAi constructs formulated for electroporation into cells in vivo. For example, the RNAi construct is formulated in supramolecular complexes or in liposomes. In certain embodiments, the cells are epithelial cells or muscle cells.

Still another aspect of the present invention provides a method for delivering one or more RNAi constructs to a patient by electroporation, comprising administering the RNAi construct of sufficient amount to an animal through electroporation, wherein the RNAi construct attenuates expression of a target gene in cells of the patient. For example, the RNAi construct of the method is formulated in supramolecular complexes or in liposomes. In certain embodiments, the cells of the method are epithelial cells or muscle cells.

In one embodiment, the present invention provides a pharmaceutical preparation comprising at least one RNAi construct formulated for electroporation into cells, and a pharmaceutically acceptable carrier. Optionally, the pharmaceutically acceptable carrier is selected from pharmaceutically acceptable salts, ester, and salts of such esters. In certain preferred embodiments, the present invention provides a pharmaceutical package comprising the pharmaceutical preparation which includes at least one RNAi formulated for electroporation into cells and a pharmaceutically acceptable carrier, in association with instructions (written and/or pictorial) for administering the preparation to a human patient.

Another aspect of the present invention provides a composition comprising one or more formulated RNAi constructs for inhibiting unwanted cell growth in vivo, wherein, through an RNA interference mechanism, the RNAi construct reduces expression of a target gene essential to mitosis of a cell and/or which is essential to preventing apoptosis of said cell.

In certain preferred embodiments, the RNAi construct of the method is an small-interfering RNA (siRNA), preferably being 19-30 base pairs in length. Alternatively, the RNAi construct is an expression vector having a coding sequence that is transcribed to produce one or more transcriptional products that produce siRNA in the treated cells. For example, the expression vector is selected from an episomal expression vector, an integrative expression vector, and a viral expression vector. In another preferred embodiment, the RNAi construct is a hairpin RNA which is processed to an siRNA in the treated cells.

In certain embodiments, the RNAi construct of the composition inhibits proliferation of the cell. Alternatively, the RNAi construct promotes apoptosis of the cell.

Exemplary RNAi constructs inhibit expression of a target gene that is an oncogene, such as c-myc, c-myb, mdm2, PKA-I, Abl-1, Bcl2, Ras, c-Raf kinase, CDC25 phosphatases, cyclins, cyclin dependent kinases, telomerase, PDGF/sis, erb-B, fos, jun, mos, src or the Bcr/Abl fusion gene.

In certain embodiments, the RNAi construct is used for the treatment of transformed cells, e.g., to inhibit or attenuate hyperplastic cell growth, and may be part of a treatment for cancer as such.

In other embodiments, the RNAi construct is used for inhibiting activation of lymphocytes, including treatment or prophylaxis of immune-mediated inflammatory disorders.

In still other embodiments, the RNAi construct is used for inhibiting proliferation of smooth muscle cells, including treatment or prophylaxis of restenosis.

In yet other embodiments, the RNAi construct is used for inhibiting proliferation of epithelial cells (e.g., as a component of cosmetic preparations).

In certain embodiments, the RNAi construct of the composition is formulated in a supramolecular complex. Optionally, the supramolecular complex comprises at least one polymer, for example, a cyclodextrin containing polymer. Alternatively, the RNAi construct is encapsulated or associated with a liposome, for example, a cationic liposome formed of a cationic vesicle-forming lipid. Optionally, the liposome complexed with the RNAi construct has a substantially homogeneous size of typically less than about 200 nm.

In a preferred embodiment, the animal is a human patient.

Still another aspect of the present invention provides a method for inhibiting unwanted cell growth in vivo, comprising administering to an animal a formulated RNAi construct of sufficient amount, wherein, through an RNA interference mechanism, the RNAi construct reduces expression of a target gene essential to mitosis of a cell and/or which is essential to preventing apoptosis of said cell.

In certain preferred embodiments, the RNA construct of the method is an small-interfering RNA (siRNA), preferably being 19-30 base pairs in length. Alternatively, the RNAi construct is an expression vector having a coding sequence that is transcribed to produce one or more transcriptional products that produce siRNA in the treated cells. For example, the expression vector is selected from an episomal expression vector, an integrative expression vector, and a viral expression vector. In another preferred embodiment, the RNAi construct is a hairpin RNA which is processed to an siRNA in the treated cells.

In certain embodiments, the RNAi construct of the composition inhibits proliferation of the cell. Alternatively, the RNAi construct promotes apoptosis of the cell.

In certain preferred embodiment, the target gene is an oncogene, such as c-myc, c-myb, mdm2, PKA-I, Abl-1, Bcl2, Ras, c-Raf kinase, CDC25 phosphatases, cyclins, cyclin dependent kinases, telomerase, PDGF/sis, erb-B, fos, jun, mos, src or the Bcr/Abl fusion gene. In certain embodiments, the cell is a transformed cell so that the RNAi construct is used for the treatment of hyperplastic cell growth, including treatment of a cancer. In other embodiments, the RNAi construct is used for inhibiting activation of lymphocytes, including treatment or prophylaxis of immune-mediated inflammatory disorders. In still other embodiments, the RNAi construct is used for inhibiting proliferation of smooth muscle cells, including treatment or prophylaxis of restenosis. In yet other embodiments, the RNAi construct is used for inhibiting proliferation of epithelial cells (e.g., as a component of cosmetic preparations).

In other embodiments, the RNAi construct of the method is formulated in a supramolecular complex. Optionally, the supramolecular complex comprises at least one polymer, for example, a cyclodextrin containing polymer.

Alternatively, the RNAi construct is encapsulated or associated with a liposome, for example, a cationic liposome formed of a cationic vesicle-forming lipid. Optionally, the liposome complexed with the RNAi construct has a substantially homogeneous size of typically less than about 200 nm.

In a preferred embodiment, the animal of the method is a human.

Another aspect of the invention provides a pharmaceutical preparation comprising at least one RNAi construct formulated for inhibiting unwanted cell growth, and a pharmaceutically acceptable carrier. Optionally, the pharmaceutically acceptable carrier is selected from pharmaceutically acceptable salts, ester, and salts of such esters. In certain preferred embodiments, the present invention provides a pharmaceutical package comprising the pharmaceutical preparation which includes at least one RNAi formulated for inhibiting unwanted cell growth and a pharmaceutically acceptable carrier, in association with instructions (written and/or pictorial) for administering the preparation to a human patient. In another embodiment, the present invention provides a cosmetic preparation comprising at least one RNAi construct formulated for inhibiting epithelial cell growth or differentiation.

Still another aspect of the present invention provides a method for inducing cell death, comprising administering to target cells in vivo an double stranded RNA, or an expression vector capable of transcribing a double stranded RNA, of sufficient length to activate a PKR response in the target cells, which double stranded RNA is formulated as part of a supramolecular complex.

In certain preferred embodiments, the double stranded RNA is more than 35 basepairs in length, and even more preferably more than 75, 100, 200 or even 400 basepairs. In certain embodiments, the target cells are mammalian cells, including transformed cells. In certain embodiments, the supramolecular complex is a multi-dimensional polymer network including linear polymers or branched polymers. Preferably, the supramolecular complex is formed from cationic polymers, such as poly(L)lysine (PLL), polyethylenimine (PEI), β-cyclodextrin containing polymers (βCD-polymers), and co-polymers thereof. 208.

In certain embodiments, the supramolecular complex is formed from cyclodextrin-modified polymers, including cyclodextrin-modified poly(ethylenimine) having a structure of the formula: 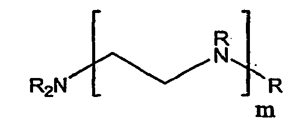 wherein
R represents, independently for each occurrence, H, lower alkyl, a cyclodextrin moiety, or 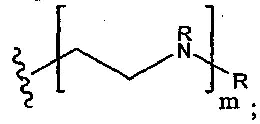 and
m, independently for each occurrence, represents an integer from 2-10,000, preferably from 10 to 5,000, or from 100 to 1,000.

Still another aspect of the present invention provides a method of conducting a pharmaceutical business comprising:
a). identifying an RNAi construct which inhibits proliferation of target cells in vivo and reduces the effects of a disorder involving unwanted proliferation of the target cells;
b). conducting therapeutic profiling of the RNAi construct identified in step (a) for efficacy and toxicity in animals; and
c). formulating a pharmaceutical preparation including one or more RNAi constructs identified in step (b) as having an acceptable therapeutic profile.

Preferably, the method of conducting a pharmaceutical business includes an additional step of establishing a distribution system for distributing the pharmaceutical preparation for sale, and (optionally) establishing a sales group for marketing the pharmaceutical preparation.

Yet still another aspect of the present invention provides a, method of conducting a pharmaceutical business comprising:
a). identifying an RNAi construct which inhibits proliferation of target cells in vivo and reduces the effects of a disorder involving unwanted proliferation of the target cells;
b). (optionally) conducting therapeutic profiling of the RNAi construct identified in step (a) for efficacy and toxicity in animal; and
c). licensing, to a third party, the rights for further development of the RNAi construct.

### Brief Description Of The Drawings

Figure 1 shows delivery of a plasmid encoding green fluorescent protein (GFP) and/or a plasmid encoding the sense and antisense strands of the siRNA oligonucleotide into HEK 293-EcR cells. Plasmid(s) were complexed with branched-PEI25k-hi-CD polymer at a ratio of 15 N/P in 0.5ml of opti-MEM for delivery into the cells. Relative GFP expression was measured after cells were transfected with the indicated plasmid(s).
**Figure 2** shows the effect of β-CD polymer 4/DNA charge ratio and serum
   conditions on transfection efficiency (• and ■) and cell survival in BHK-21 cells.
   Results from transfections in 10% serum and serum-free media are shown with the dotted and solid lines, respectively. Data are reported as the mean +/- SD. of three
   samples.

### Detailed Description of the Invention

### I. Overview

The present invention provides methods and compositions for attenuating expression of a target gene *in vivo.* In general, the method includes administering RNAi constructs (such as small-interfering RNAs (i.e., siRNAs) that are targeted to particular mRNA sequences, or nucleic acid material that can produce siRNAs in a cell), in an amount sufficient to attenuate expression of a target gene by an RNA interference mechanism, e.g., in a sequence-dependent, PKR-independent manner. In particular, the subject method can be used to alter the growth, survival or differentiation of cells for therapeutic and cosmetic purposes.

One aspect of the invention relates to the use ofRNAi constructs to attenuate expression of proliferation-regulating genes (including apoptosis-inhibiting genes). Such embodiments can be used as part of a therapeutic or cosmetic treatment program to inhibit, or at least reduce, unwanted growth of cells *in vivo,* and particularly the growth of transformed cells.

Another aspect of invention relates to formulations of RNAi constructs for pulmonary administration, e.g., respirable RNAi constructs. Such formulations can be used for local or systemic delivery of RNAi constructs and constitute a convenient method for administration of RNAi constructs for any of a variety of indications, e.g., not limited to treating proliferative disorders. Merely to illustrate, certain RNAi compositions of the subject invention can be used to knockdown expression of vasoconstrictors, or reduce receptor levels of the vasoconstrictors, to reduce blood pressure in patients suffering from systemic and pulmonary hypertension.

Yet another aspect of the invention relates to methods of treating patients with RNA constructs through percutaneous intrapericardial drug delivery in which the pericardial space is effectively used as a delivery reservoir for RNAi constructs. While useful in systemic delivery of RNAi constructs, it is contemplated that such techniques can be especially useful for local delivery to the heart and surrounding vasculature.

For instance, in the treatments of myocardial infarction, the invention provides a method for administering angiogenic RNAi constructs to promote angiogenesis and thereby promote recovery and/or prevent further damage to the tissue in an around the infarct. For instance, the subject method can be used to deliver an RNAi construct which inhibits expression of a protein that negatively regulates the activity of the NF-κB transcription factor, such as by inhibiting expressing of NF-κB repressor IκB, or any other cellular factor which reduces basic fibroblast growth factor-induced angiogenesis *in vivo.* Other targets for RNAi-mediated attenuation include the gene encoding C-reactive protein (CRP). CRP inhibits both basal and vascular endothelial growth factor-stimulated angiogenesis.

Intrapericardial drug delivery can also be used for delivery of RNAi constructs which reduce proliferation and/or migration smooth muscle cells and thereby may be useful in treating neointimal hyperplasia, such as restenosis, artherosclerosis and the like. Merely to illustrate, inhibition of neointimal hyperplasia can be achieved by administration of RNAi constructs for attenuating gene expression of c-myb, c-myc, proliferating cell nuclear antigen (PCNA), transforming growth factor-beta (TGF-beta), and transcription factors such as nuclear factor kappaB (NF-κB) and E2F. In addition to intrapericardial drug delivery for reducing neointimal hyperplasia, the present invention also specifically contemplates the delivery of RNAi constructs "on stent", either by directly coating at least a portion of the stent with RNAi constructs, or through a polymeric coating from which the RNAi constructs are released.

Another aspect of the invention relates to coated medical devices. For instance, in certain embodiments, the subject invention provides a medical device having a coating adhered to at least one surface, wherein the coating includes the subject polymer matrix and an RNAi construct. Such coatings can be applied to surgical implements such as screws, plates, washers, sutures, prosthesis anchors, tacks, staples, electrical leads, valves, membranes. The devices can be catheters, implantable vascular access ports, blood storage bags, blood tubing, central venous catheters, arterial catheters, vascular grafts, intraaortic balloon pumps, heart valves, cardiovascular sutures, artificial hearts, a pacemaker, ventricular assist pumps, extracorporeal devices, blood filters, hemodialysis units, hemoperfasion units, plasmapheresis units, and filters adapted for deployment in a blood vessel.

Still another aspect of the invention provides compositions of RNAi constructs suitable for electroporation into cells *in vivo,* such as electroporation into epithelial tissues (skin, mucosal membranes and the like) as well as into muscle (smooth or skeletal).

Another aspect of the invention, RNAi constructs are formulated in a supramolecular complex, and are suitable for use as a pharmaceutical agent, e.g., substantially free of pyrogenic agents. For instance, the supramolecular complex can be formed from a multi-dimensional polymer network comprising a linear polyethyleneimine or a linear cyclodextrin-containing polymer and a branched polyethyleneimine or branched cyclodextrin polymer. In certain preferred embodiments, the expression constructs are formulated with cyclodextrins, e.g., such as a cyclodextrin cellular delivery system.

Yet another aspect of the invention relates to the use of long double stranded RNA to activate the sequence independent dsRNA response in certain cells, e.g., to activate dsRNA-dependent protein kinase PKR. PKR-mediated response to long dsRNA (e.g., dsRNA greater than 35 basepairs, and even more preferably greater than 39,50, 75,100 or even 200 basepairs) is a potent growth inhibitory protein that is primarily activated in virally infected cells, inducing cell death. The subject compositions described herein for carrying out sequence-dependent RNA interference can be readily adapted to deliver long dsRNA (e.g., dsRNA molecule of sufficient length to activate PKR and induce cell death, such as in the range of 40-1000 basepairs, preferably 100-800 basepairs, and even more preferably 200-500 basepairs) to cells for which it is desired that cell death occur. In one embodiment, the subject long dsRNA formulations can be used to kill cancer cells.

### 11 Definitions

For convenience, certain terms employed in the specification, examples, and appended claims are collected here.

As used herein the term "animal" refers to mammals, preferably mammals such as humans. Likewise, a "patient" or "subject" to be treated by the method of the invention can mean either a human or non-human animal.

The terms "apoptosis" or "programmed cell death," refers to the physiological process by which unwanted or useless cells are eliminated during development and other normal biological processes. Apoptosis, is a mode of cell death that occurs under normal physiological conditions and the cell is an active participant in its own demise ("cellular suicide"). It is most often found during normal cell turnover and tissue homeostasis, embryogenesis, induction and maintenance of immune tolerance, development of the nervous system and endocrine-dependent tissue atrophy. Cells undergoing apoptosis show characteristic morphological and biochemical features. These features include chromatin aggregation, nuclear and cytoplasmic condensation, partition of cytoplasm and nucleus into membrane bound vesicles (apoptotic bodies) which contain ribosomes, morphologically intact mitochondria and nuclear material. *In vivo,* these apoptotic bodies are rapidly recognized and phagocytized by either macrophages or adjacent epithelial cells. Due to this efficient mechanism for the removal of apoptotic cells *in vivo* no inflammatory response, is elicited. *In vitro,* the apoptotic bodies as well as the remaining cell fragments ultimately swell and finally lyse. This terminal phase of *in vitro* cell death has been termed "secondary necrosis."

"Cells," "host cells" or "recombinant host cells" are terms used interchangeably herein. It is understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

A "disease-associated" or "disease-causing" gene refers to any gene the expression of which is essential to or substantially contributes an unwanted cellular phenotype. It may be a gene that becomes expressed at an abnormally high level; it maybe a gene that becomes expressed at an abnormally low level, where the altered expression correlates with the occurrence and/or progression of the disease. A disease-associated gene also refers to gene possessing mutation(s) or genetic variation that is directly responsible or is in linkage disequilibrium with gene(s) that is responsible for the etiology of a disease. The transcribed or translated products may be known or unknown, and may be at normal or abnormal level.

As used herein, the term "dsRNA" refers to siRNA molecules, or other RNA molecules including a double stranded feature and able to be processed to siRNA in cells, such as hairpin RNA moieties.

Likewise, the term "encodes," unless evident from its context, will be meant to include DNA sequences that encode a polypeptide, as the term is typically used, as well as DNA sequences that are transcribed into inhibitory antisense molecules.

The term "expression" with respect to a gene sequence refers to transcription of the gene and, as appropriate, translation of the resulting mRNA transcript to a protein. Thus, as will be clear from the context, expression of a protein coding sequence results from transcription and translation of the coding sequence.

The "growth state" of a cell refers to the rate of proliferation of the cell and the state of differentiation of the cell.

As used herein, "immortalized cells" refers to cells that have been altered via chemical, genetic, and/or recombinant means such that the cells have the ability to grow through an indefinite number of divisions in culture.

"Inhibition of gene expression refers to the absence (or observable decrease) in the level of protein and/or mRNA product from a target gene. "Specificity" refers to the ability to inhibit the target gene without manifest effects on other genes of the cell. The consequences of inhibition can be confirmed by examination of the outward properties of the cell or organism (as presented below in the examples) or by biochemical techniques such as RNA solution hybridization, nuclease protection, Northern hybridization, reverse transcription, gene expression monitoring with a microarray, antibody binding, enzyme linked immunosorbent essay (ELISA), Western blotting, radiolmmunoassay (RIA), other immunoassays, and fluorescence activated cell analysis (FACS).

The term "loss-of-function,' as it refers to genes inhibited by the subject RNAi method, refers a diminishment in the level of expression of a gene when compared to the level in the absence of RNAi constructs.

As used herein, the phrase "mediates RNAi" refers to (indicates) the ability to distinguish which RNAs are to be degraded by the RNAi process, e.g., degradation occurs in a sequence-specific manner rather than by a sequence-independent dsRNA response, e.g., a PKR response.

The term "nasal delivery" refers to systemic delivery of RNAi constructs to a patient by inhalation through and into the nose.

As used herein, the term "nucleic acid" refers to polynucleotides such as deoxyribonucleic acid (DNA), and, where appropriate, ribonucleic acid (RNA). The term should also be understood to include, as applicable to the embodiment being described, single-stranded (such as sense or antisense) and double-stranded polynucleotides.

"Operably linked" when describing the relationship between two DNA regions simply means that they are functionally related to each other. For example, a promoter or other transcriptional regulatory sequence is operably linked to a coding sequence if it controls the transcription of the coding sequence.

The term "pharmaceutically acceptable salts" refers to physiologically and pharmaceutically acceptable salts of the compounds of the invention, i.e., salts that retain the desired biological activity of the parent compound and do not impart undesired toxicological effects thereto.

As used herein, "proliferating" and "proliferation" refer to cells undergoing mitosis.

A "protein coding sequence" or a sequence that "encodes" a particular polypeptide or peptide, is a nucleic acid sequence that is transcribed (in the case of DNA) and is translated (in the case of mRNA) into a polypeptide *in vitro* or *in vivo* when placed under the control of appropriate regulatory sequences. The boundaries of the coding sequence are determined by a start codon at the 5' (amino) terminus and a translation stop codon at the 3' (carboxyl) terminus. A coding sequence can include, but is not limited to, cDNA from prokaryotic or eukaryotic mRNA, genomic DNA sequences from prokaryotic or eukaryotic DNA, and even synthetic DNA sequences. A transcription termination sequence will usually be located 3' to the coding sequence.

The terms "pulmonary delivery" and "respiratory delivery" refer to systemic delivery of RNAi constructs to a patient by inhalation through the mouth and into the lungs.

By "recombinant virus" is meant a virus that has been genetically altered, e.g., by the addition or insertion of a heterologous nucleic acid construct into the particle.

As used herein, the term "RNAi construct" is a generic term used throughout the specification to include small interfering RNAs (siRNAs), hairpin RNAs, and other RNA species which can be cleaved *in vivo* to form siRNAs. RNAi constructs herein also include expression vectors (also referred to as RNAi expression vectors) capable of giving rise to transcripts which form dsRNAs or hairpin RNAs in cells, and/or transcripts which can produce siRNAs *in vivo*.

"RNAi expression vector" (also referred to herein as a "dsRNA-encoding plasmid") refers to a replicable nucleic acid constructs used to express (transcribe) RNA which produces siRNA moieties in the cell in which the construct is expressed. Such vectors include a transcriptional unit comprising an assembly of (1) genetic element(s) having a regulatory role in gene expression, for example, promoters, operators, or enhancers, operatively linked to (2) a "coding" sequence which is transcribed to produce a double-stranded RNA (two RNA moieties that anneal in the cell to form an siRNA, or a single hairpin RNA which can be processed to an siRNA), and (3) appropriate transcription initiation and termination sequences. The choice of promoter and other regulatory elements generally varies according to the intended host cell. In general, expression vectors of utility in recombinant DNA techniques are often in the form of "plasmids" which refer to circular double stranded DNA loops which, in their vector form are not bound to the chromosome. In the present specification, "plasmid" and "vector" are used interchangeably as the plasmid is the most commonly used form of vector. However, the invention is intended to include such other forms of expression vectors which serve equivalent functions and which become known in the art subsequently hereto.

In the expression vectors, regulatory elements controlling transcription can be generally derived from mammalian, microbial, viral or insect genes. The ability to replicate in a host, usually conferred by an origin of replication, and a selection gene to facilitate recognition of transformants may additionally be incorporated. Vectors derived from viruses, such as retroviruses, adenoviruses, and the like, may be employed.

The term "small interfering RNAs" or "siRNAs" refers to nucleic acids around 19-30 nucleotides in length, and more preferably 21-23 nucleotides in length. The siRNAs are double-stranded, and may include short overhangs at each end. Preferably, the overhangs are 1-6 nucleotides in length at the 3' end. It is known in the art that the siRNAs can be chemically synthesized, or derive from a longer double-stranded RNA or a hairpin RNA. The siRNAs have significant sequence similarity to a target RNA so that the siRNAs can pair to the target RNA and result in sequence-specific degradation of the target RNA through an RNA interference mechanism. Optionally, the siRNA molecules comprise a 3' hydroxyl group.

The term "supramolecular complex" refers to a multi-dimensional polymer network formed with at least one polymer. The polymer molecule may be linear or branched, for example, poly(L)lysine (PLL), polyethylenimine (PEI), β-cyclodextrin containing polymers (βCD-polymers), and co-polymers thereof. In certain embodiments, the present invention relates to RNAi constructs formulated as supramolecular complexes.

"Transcriptional regulatory sequence" is a generic term used throughout the specification to refer to. DNA sequences, such as initiation signals, enhancers, and promoters and the like which induce or control transcription of coding sequences with which they are operably linked.

As used herein, the terms "transduction" and "transfection" are art recognized and mean the introduction of a nucleic acid, e.g., an expression vector, into a recipient cell by nucleic acid-mediated gene transfer. "Transformation," as used herein, refers to a process in which a cell's genotype is changed as a result of the cellular uptake of exogenous DNA or RNA, and, for example, the transformed cell expresses an RNAi construct. A cell has been "stably transfected" with a nucleic acid construct when the nucleic acid construct is capable of being inherited by daughter cells.

As used herein, "transformed cells" refers to cells that have spontaneously converted to a state of unrestrained growth, i.e., they have acquired the ability to grow through an indefinite number of divisions in culture. Transformed cells may be characterized by such terms as neoplastic, anaplastic and/or hyperplastic, with respect to their loss of growth control. For purposes of this invention, the terms "transformed phenotype of malignant mammalian cells" and "transformed phenotype " are intended to encompass, but not be limited to, any of the following phenotypic traits associated with cellular transformation of mammalian cells: immortalization, morphological or growth transformation, and tumorigenicity, as detected by prolonged growth in cell culture, growth in semi-solid media, or tumorigenic growth in immuno-incompetent or syngeneic animals.

"Transient transfection" refers to cases where exogenous DNA does not integrate into the genome of a transfected cell, e.g., where episomal DNA is transcribed into mRNA and translated into protein.

As used herein, the term "vector" refers to a nucleic acid molecule capable of transporting another nucleic acid to that it has been linked. One type of vector is a genomic integrated vector, or "integrated vector," which can become integrated into the chromosomal DNA of the host cell. Another type of vector is an episomal vector, i.e., a nucleic acid capable of extra-chromosomal replication. Vectors capable of directing the expression of genes to that they are operatively linked are referred to herein as "expression vectors." In the present specification, "plasmid" and "vector" are used interchangeably unless otherwise clear from the context.

### III. Exemplary RNAi constructs

The RNAi constructs contain a nucleotide sequence that hybridizes under physiologic conditions of the cell to the nucleotide sequence of at least a portion of the mRNA transcript- for the gene to be inhibited (i.e., the "target" gene). The double-stranded RNA need only be sufficiently similar to natural RNA that it has the ability to mediate RNAi. Thus, the invention has the advantage of being able to tolerate sequence variations that might be expected due to genetic mutation, strain polymorphism or evolutionary divergence. The number of tolerated nucleotide mismatches between the target sequence and the RNAi construct sequence is no more than 1 in 5 basepairs, or 1 in 10 basepairs, or 1 in 20 basepairs, or 1 in 50 basepairs. Mismatches in the center of the siRNA duplex are most critical and may essentially abolish cleavage of the target RNA. In contrast, nucleotides at the 3' end of the siRNA strand that is complementary to the target RNA do not significantly contribute to specificity of the target recognition.

Sequence identity may be optimized by sequence comparison and alignment algorithms known in the art (see Gribskov and Devereux, Sequence Analysis Primer, Stockton Press, 1991, and references cited therein) and calculating the percent difference between the nucleotide sequences by, for example, the Smith-Waterman algorithm as implemented in the BESTFIT software program using default parameters (e.g., University of Wisconsin Genetic Computing Group). Greater than 90% sequence identity, or even 100% sequence identity, between the inhibitory RNA and the portion of the target gene is preferred. Alternatively, the duplex region of the RNA may be defined functionally as a nucleotide sequence that is capable of hybridizing with a portion of the target gene transcript (e.g., 400 mM NaCl, 40 mM PIPES pH 6.4,1 mM EDTA, 50 °C or 70 °C hybridization for 12-16 hours; followed by washing).

Production of RNAi constructs can be carried out by chemical synthetic methods or by recombinant nucleic acid techniques. Endogenous RNA polymerase of the treated cell may mediate transcription *in vivo,* or cloned RNA polymerase can be used for transcription *in vitro.* The RNAi constructs may include modifications to either the phosphate-sugar backbone or the nucleoside, e.g., to reduce susceptibility to cellular nucleases, improve bioavailability, improve formulation characteristics, and/or change other pharmacokinetic properties. For example, the phosphodiester linkages of natural RNA may be modified to include at least one of an nitrogen or sulfur heteroatom. Modifications in RNA structure may be tailored to allow specific genetic inhibition while avoiding a general response to dsRNA. Likewise, bases may be modified to block the activity of adenosine deaminase. The RNAi construct may be produced enzymatically or by partial/total organic synthesis, any modified ribonucleotide can be introduced by *in vitro* enzymatic or organic synthesis.

Methods of chemically modifying RNA molecules can be adapted for modifying RNAi constructs (see, for example, Heidenreich et al. (1997) Nucleic Acids Res. 25:776-780; Wilson et al. (1994) J Mol Recog 7:89-98; Chen et al. (1995) Nucleic Acids Res 23:2661-2668; Hirschbein et al. (1997) Antisense Nucleic Acid Drug Dev 7:55-61). Merely to illustrate, the backbone of an RNAi construct can be modified with phosphorothioates, phosphoramidate, phosphodithioates, chimeric methylphosphonate-phosphodiesters, peptide nucleic acids, 5-propynyl-pyrimidine containing oligomers or sugar modifications (e.g., 2'-substituted ribonucleosides, α-configuration).

The double-stranded structure may be formed by a single self-complementary RNA strand or two complementary RNA strands. RNA duplex formation may be initiated either inside or outside the cell. The RNA may be introduced in an amount which allows delivery of at least one copy per cell. Higher doses (e.g., at least 5, 10, 100, 500 or 1000 copies per cell) of double-stranded material may yield more effective inhibition, while lower doses may also be useful for specific applications. Inhibition is sequence-specific in that nucleotide sequences corresponding to the duplex region of the RNA are targeted for genetic inhibition.

In certain embodiments, the subject RNAi constructs are "small interfering RNAs" or "siRNAs." These nucleic acids are around 19-30 nucleotides in length, and even more preferably 21-23 nucleotides in length, e.g., corresponding in length to the fragments generated by nuclease "dicing" of longer double-stranded RNAs. The siRNAs are understood to recruit nuclease complexes and guide the complexes to the target mRNA by pairing to the specific sequences. As a result, the target mRNA is degraded by the nucleases in the protein complex. In a particular embodiment, the 21-23 nucleotides siRNA molecules comprise a 3' hydroxyl group.

The siRNA molecules of the present invention can be obtained using a number of techniques known to those of skill in the art. For example, the siRNA can be chemically synthesized or recombinantly produced using methods known in the art. For example, short sense and antisense RNA oligomers can be synthesized and annealed to form double-stranded RNA structures with 2-nucleotide overhangs at each end (Caplen, et al. (2001) Proc Natl Acad Sci USA, 98:9742-9747; Elbashir, et al. (2001) EMBO J, 20:6877-88). These double-stranded siRNA structures can then be directly introduced to cells, either by passive uptake or a delivery system of choice, such as described below.

In certain embodiments, the siRNA constructs can be generated by processing of longer double-stranded RNAs, for example, in the presence of the enzyme dicer. In one embodiment, the Drosophila *in vitro* system is used. In this embodiment, dsRNA is combined with a soluble extract derived from Drosophila embryo, thereby producing a combination. The combination is maintained under conditions in which the dsRNA is processed to RNA molecules of about 21 to about 23 nucleotides.

The siRNA molecules can be purified using a number of techniques known to those of skill in the art. For example, gel electrophoresis can be used to purify siRNAs. Alternatively, non-denaturing methods, such as non-denaturing column chromatography, can be used to purify the siRNA. In addition, chromatography (e.g., size exclusion chromatography), glycerol gradient centrifugation, affinity purification with antibody can be used to purify siRNAs.

In certain preferred embodiments, at least one strand of the siRNA molecules has a 3' overhang from about 1 to about 6 nucleotides in length, though may be from 2 to 4 nucleotides in length. More preferably, the 3' overhangs are 1-3 nucleotides in length. In certain embodiments, one strand having a 3' overhang and the other strand being blunt-ended or also having an overhang. The length of the overhangs may be the same or different for each strand. In order to further enhance the stability of the siRNA, the 3' overhangs can be stabilized against degradation. In one embodiment, the RNA is stabilized by including purine nucleotides, such as adenosine or guanosine nucleotides. Alternatively, substitution of pyrimidine nucleotides by modified analogues, e.g., substitution of uridine nucleotide 3' overhangs by 2'-deoxythyinidine is tolerated and does not affect the efficiency of RNAi. The absence of a 2' hydroxyl significantly enhances the nuclease resistance of the overhang in tissue culture medium and may be beneficial *in vivo.*

In other embodiments, the RNAi construct is in the form of a long double-stranded RNA. In certain embodiments, the RNAi construct is at least 25, 50, 100, 200, 300 or 400 bases. In certain embodiments, the RNAi construct is 400-800 bases in length. The double-stranded RNAs are digested intracellularly, e.g., to produce siRNA sequences in the cell. However, use of long double-stranded RNAs *in vivo* is not always practical, presumably because of deleterious effects which may be caused by the sequence-independent dsRNA response. In such embodiments, the use of local delivery systems and/or agents which reduce the effects of interferon or PKR are preferred.

In certain embodiments, the RNAi construct is in the form of a hairpin structure (named as hairpin RNA). The hairpin RNAs can be synthesized exogenously or can be formed by transcribing from RNA polymerase III promoters *in vivo:* Examples of making and using such hairpin RNAs for gene silencing in mammalian cells are described in, for example, Paddison et al., Genes Dev. 2002, 16:948-58; McCaffrey et al., Nature. 2002, 418:38-9; McManus et al., RNA. 2002, 8:842-50; Yu et al., Proc Natl Acad Sci U S A. 2002, 99:6047-52). Preferably, such hairpin RNAs are engineered in cells or in an animal to ensure continuous and stable suppression of a desired gene. It is known in the art that siRNAs can be produced by processing a hairpin RNA in the cell.

In yet other embodiments, a plasmid is used to deliver the double-stranded RNA, e.g., as a transcriptional product. In such embodiments, the plasmid is designed to include a "coding sequence" for each of the sense and antisense strands of the RNAi construct. The coding sequences can be the same sequence, e.g., flanked by inverted promoters, or can be two separate sequences each under transcriptional control of separate promoters. After the coding sequence is transcribed, the complementary RNA transcripts base-pair to form the double-stranded RNA.

PCT application WO01/77350 describes an exemplary vector for bidirectional transcription of a transgene to yield both sense and antisense RNA transcripts of the same transgene in a eukaryotic cell. Accordingly, in certain embodiments, the present invention provides a' recombinant vector having the following unique characteristics: it comprises a viral replicon having two overlapping transcription units arranged in an opposing orientation and flanking a transgene for an RNAi construct of interest, wherein the two overlapping transcription units yield both sense and antisense RNA transcripts from the same transgene fragment in a host cell.

### IV. Exemplary Formulations

The RNAi constructs of the invention may also be admixed, encapsulated, conjugated or otherwise associated with other molecules, molecule structures or mixtures of compounds, as for example, liposomes, polymers, receptor targeted molecules, oral, rectal, topical or other formulations, for assisting in uptake, distribution and/or absorption. The subject RNAi constructs can be provided in formulations also including penetration enhancers, carrier compounds and/or transfection agents.

Representative United States patents that teach the preparation of such uptake, distribution and/or absorption assisting formulations which can be adapted for delivery of RNAi constructs, particularly siRNA molecules, include, but are not limited to, U.S. 5,108,921; 5,354,844; 5,416,016; 5,459,127; 5,521,291;51543,158; 5,547,932; 5,583,020; 5,591,721; 4,426,330;4,534,899; 5,013,556; 5,108,921; 5,213,804; 5,227,170;5,264,221; 5,356,633; 5,395,619; 5,416,016; 5,417,978;5,462,854; 5,469,854; 5,512,295; 5,527,528; 5,534,259; 5,543,152; 5,556,948; 5,580,575; and 5,595,756.

The RNAi constructs of the invention also encompass any pharmaceutically acceptable salts, esters or salts of such esters, or any other compound which, upon administration to an animal including a human, is capable of providing (directly or indirectly) the biologically active metabolite or residue thereof. Accordingly, for example, the disclosure is also drawn to RNAi constructs and pharmaceutically acceptable salts of the siRNAs, pharmaceutically acceptable salts of such RNAi constructs, and other bioequivalents.

Pharmaceutically acceptable base addition salts are formed with metals or amines, such as alkali and alkaline earth metals or organic amines. Examples of metals used as cations are sodium, potassium, magnesium, calcium, and the like. Examples of suitable amines are N,NI-dibenzylethylenediamine, chloroprocaine, choline, diethanolamine, dicyclohexylamine, ethylenediamine, N-methylglucamine, and procaine (see, for example, Berge et al., "Pharmaceutical Salts," J. of Pharma Sci.,1977, 66,1-19). The base addition salts of said acidic compounds are prepared by contacting the free acid form with a sufficient amount of the desired base to produce the salt in the conventional manner. The free acid form may be regenerated by contacting the salt form with an acid and isolating the free acid in the conventional manner. The free acid forms differ from their respective salt forms somewhat in certain physical properties such as solubility in polar solvents, but otherwise the salts are equivalent to their respective free acid for purposes of the present invention. As used herein, a "pharmaceutical addition salt" includes a pharmaceutically acceptable salt of an acid form of one of the components of the compositions of the invention. These include organic or inorganic acid salts of the amines. Preferred acid salts are the hydrochlorides, acetates, salicylates, nitrates and phosphates. Other suitable pharmaceutically acceptable salts are well known to those skilled in the art and include basic salts of a variety of inorganic and organic acids.

For siRNA oligonucleotides, preferred examples of pharmaceutically acceptable salts include but are not limited to (a) salts formed with cations such as sodium, potassium, ammonium, magnesium, calcium, polyamines such as spermine and spermidine, etc.; (b) acid addition salts formed with inorganic acids, for example hydrochloric acid, hydrobromic acid, sulfuric acid, phosphoric acid, nitric acid and the like; (c) salts formed with organic acids such as, for example, acetic acid, oxalic acid, tartaric acid, succinic acid, maleic acid, fumaric acid, gluconic acid, citric acid, malic acid, ascorbic acid, benzoic acid, tannic acid, palmitic acid, alginic acid, polyglutamic acid, naphthalenesulfonic acid, methanesulfonic acid, p-toluenesulfonic acid, naphthalene disulfonic acid, polygalacturonic acid, and the like; and (d) salts formed from elemental anions such as chlorine, bromine, and iodine.

### A. Supramolecular complexes

In certain embodiments, the subject RNAi constructs are formulated as part of a "supramolecular complex." To further illustrate, the RNAi construct can be contacted with at least one polymer to form a composite and then the polymer of the composite treated under conditions sufficient to form a supramolecular complex containing the RNAi construct and a multi-dimensional polymer network. The polymer molecule may be linear or branched. Accordingly, a group of two or more polymer molecules may be linear, branched, or a mixture of linear and branched polymers. The composite may be prepared by any suitable means known in the art. For example, the composite may be formed by simply contacting, mixing or dispersing the RNAi construct with a polymer. A composite may also be prepared by polymerizing monomers, which may be the same or different, capable of forming a linear or branched polymer in the presence of the expression construct. The composite may be further modified with at least one ligand, e.g., to direct cellular uptake of the expression construct or otherwise effect tissue or cellular distribution *in vivo* of the expression construct. The composite may take any suitable form and, preferably, is in the form of particles.

In certain preferred embodiments, the subject RNAi constructs are formulated with cationic polymers. Exemplary cationic polymers include poly(L)lysine (PLL) and polyethylenimine (PEI). In certain preferred embodiments, the subject expression constructs are formulated with β-cyclodextrin containing polymers (βCD-polymers). βCD-polymers are capable of forming polyplexes with nucleic acids and transfecting cultured cells. The βCD-polymers can be synthesized, for instance, by the condensation of a diamino-cyclodextrin monomer A with a diimidate comonomer B. Cyclodextrins are cyclic polysaccharides containing naturally occurring D(+)-glucopyranose units in an α-(1,4) linkage. The most common cyclodextrins are α-cyclodextrins, α-cyclodextrins and γ-cyclodextrins which contain, respectively, six, seven or eight glucopyranose units. Exemplary cyclodextrin delivery systems which can be readily adapted for delivery of the subject RNAi constructs are described in, for example, the Gonzalez et al PCT application WO00/01734 and Davis PCT application WO00/33885.

In certain embodiments, the supramolecular complexes are aggregated into particles, for example, formulations of particles having an average diameter of between 20 and 500 nanometer (nm), and even more preferably, between 20 and 200 nm.

### B. Other cationic, non-lipid formulations

In certain embodiments, the RNAi constructs are provided in cationic, non-lipid vehicles and formulated to be used in aerosol delivery via the respiratory tract. Formulations using poly(ethyleneimine) and macromolecules such as dsRNA and dsRNA-encoding plasmids can result in a high level of pulmonary transfection and increased stability during nebulization. PEI-nucleic acid formulations can also exhibit a high degree of specificity for the lungs.

In addition to formulating RNAi constructs with PEI, the invention also contemplates the use of cyclodextrin-modified polymers, such as cyclodextrin-modified poly(ethylenimine). In certain embodiments, the subject polymers have a structure of the formula: 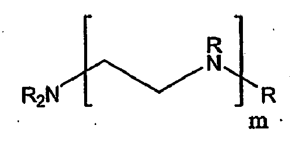 wherein R represents, independently for each occurrence, H, lower alkyl, a cyclodextrin moiety, or 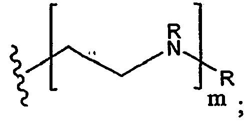 and
m, independently for each occurrence, represents an integer from 2-10,000, preferably from 10 to 5,000, or from 100 to 1,000.

In certain embodiments, R represents a cyclodextrin moiety for at least about 1%, more preferably at least about 2%, or at least about 3%, and up to about 5% or even 10%, of the nitrogen atoms that would be primary amines (i.e., bearing two occurrences ofR that represent H) but for the cyclodextrin moieties.

In certain embodiments, the cyclodextrin moieties make up at least about 2%, 3% or 4% by weight, up to 5%, 7%, or even 10% of the cyclodextrin-modified polymer by weight.

In certain embodiments, at least about 2%, 3% or 4% by weight, up to 5%, 7%, or even 10% of the ethylenimine subunits in the polymer are modified with a cyclodextrin moiety.

Copolymers of poly(ethylenimine) that bear nucleophilic amino substituents susceptible to derivatization with cyclodextrin moieties can also be used to prepare cyclodextrin-modified polymers within the scope of the present invention.

Exemplary cyclodextrin moieties include cyclic structures consisting essentially of from 7 to 9 saccharide moieties, such as cyclodextrin and oxidized cyclodextrin. A cyclodextrin moiety optionally comprises a linker moiety that forms a covalent linkage between the cyclic structure and the polymer backbone, preferably having from 1 to 20 atoms in the chain, such as alkyl chains, including dicarboxylic acid derivatives (such as glutaric acid derivatives, succinic acid derivatives, and the like), and heteroalkyl chains, such as oligoethylene glycol chains.

### C. Liposome Formulations

In certain embodiments, the invention provides composition including dsRNA or dsRNA-encoding plasmids that are encapsulated or otherwise associated with liposomes. Merely to illustrate, dsRNA moieties or dsRNA-encoding plasmids can be condensed with a polycationic condensing agent, suspended in a low-ionic strength aqueous medium, and cationic liposomes formed of a cationic vesicle-forming lipid. The ratio of liposome lipids to plasmid can be adjusted achieving maximum transfection. That ratio, in nmole liposome lipid/µg plasmid, will often be greater than 5 but less than 25, and preferably greater than 8 but less than 18, and more preferably greater than 10 but less than 15 and most preferably between 12-14. Such complexes preferably have a substantially homogeneous size (i.e., ± 20%, preferably ± 10% or more preferably ± 5% in size) of typically less than about 200 nm and preferably in the range of 50-200 nm.

Liposomes, as used herein, refer to lipid vesicles having an outer lipid shell, typically formed on one or more lipid bilayers, encapsulating an aqueous interior. In a preferred embodiment, the liposomes are cationic liposomes composed of between about 20-80 mole percent of a cationic vesicle-forming lipid, with the remainder neutral vesicle-forming lipids and/or other components. As used herein, "vesicle-forming lipid" refers to any amphipathic lipid having hydrophobic and polar head group moieties and which by itself can form spontaneously into bilayer vesicles in water, as exemplified by phospholipids. A preferred vesicle-forming lipid is a diacyl-ohain lipid, such as a phospholipid, whose acyl chains are typically between about 14-22 carbon atoms in length, and have varying degrees ofunsaturation.

A cationic vesicle-forming lipid is one whose polar head group with a net positive charge, at the operational pH, e.g., pH 4-9. Typical examples include phospholipids, such as phosphatidylethanolamine, whose polar head groups are derivatized with a positive moiety, e.g., lysine, as illustrated, for example, for the lipid DOPE derivatized with L-lysine (LYS-DOPE) (Guo, et al., 1993). Also included in this class are the glycolipids, such as cerebrosides and gangliosides having a cationic polar head-group.

Another cationic vesicle-forming lipid which may be employed is cholesterol amine and related cationic sterols. Exemplary cationic lipids include 1,2-diolelyloxy-3-(trimethylamino) - propane (DOTAP); N-[1-(2,3,-ditetradecyloxy)propyl]-N,N-dimethyl-N-hydroxyethylammonium bromide (DMR1E); N-[1-(2,3,-dioleyloxy)propyl]-N,N-dimethyl-N-hydroxy ethylammonium bromide (DORIE); N-[1-(2,3-dioleyloxy) propyl]-N,N,N-trimethylammonium chloride (DOTMA); 3β [N-(N',N'-dimethylaminoethane) carbamoly] cholesterol (DC-Chol); and dimethyldioctadecylammonium (DDAB).

The remainder of the liposomes are formed of neutral vesicle-forming lipids, meaning vesicle forming lipids which have no net charge or which may include a small percentage of lipids having a negative charge in the polar head group. Included in this class of lipids are the phospholipids, such as phosphatidylcholine (PC), phosphatidyl ethanolamine (PE), phosphatidylinositol (PI), and sphingomyelin (SM), and cholesterol, cholesterol derivatives, and other uncharged sterols.

The above-described lipids can be obtained commercially, or prepared according to published methods. Other lipids that can be included in the invention are glycolipids, such as cerebrosides and gangliosides.

In one embodiment of the invention, the dsRNA or dsRNA-encoding plasmid-liposome complex includes liposomes having a surface coating of hydrophilic polymer chains, effective to extend the blood circulation time of the plasmid/liposome complexes. Suitable hydrophilic polymers include cyclodextrin (CD), polyethylene glycol (PEG), polylactic acid, polyglycolic acid, polyvinyl-pyrrolid-one, polymethyloxazoline, polyethyloxazoline, polyhydroxypropyl methacrylamide, polymethacrylamide, polydimethylacrylamide, and derivatized celluloses, such as hydroxymethylcellulose or hydroxyethyl-cellulose. A preferred hydrophilic polymer chain is polyethyleneglycol (PEG), preferably as a PEG chain having a molecular weight between 500-10,000 daltons, more preferably between 1,000-5,000 daltons. The hydrophilic polymer may have solubility in water and in a non-aqueous solvent, such as chloroform.

The coating is preferably prepared by including in the vesicle-forming lipids a phospholipid or other diacyl-chain lipid, derivatized at its head group with the polymer chain. Exemplary methods of preparing such lipids, and forming polymer coated liposomes therewith, have been described in U.S. Pat. Nos. 5,013,556, and 5,395,619, which are incorporated herein by reference.

It will be appreciated that the hydrophilic polymer can be stably coupled to the lipid, or coupled through an unstable linkage which allows the polymer-coated plasmid-liposome complexes to shed or "release" the hydrophilic polymer coating during circulation in the bloodstream or after localization at a target site. Attachments of hydrophilic polymers, in particular polyethyleneglycol (PEG), to vesicle-forming lipids through a bond effective to release the polymer chains in response to a stimulus have been described, for example in WO 98/16202, WO 98/16201, which are hereby incorporated by reference, and by Kirpotin, D. et a. (FEBS Letters, 388:115-118 (1996).

The releasable linkage, in one embodiment, is a chemically releasable linkage which is cleaved by administration of a suitable releasing agent or is cleaved under selective physiological conditions, such as in the presence of enzymes or reducing agents. For example, ester and peptide linkages are cleaved by esterase or peptidase enzymes. Disulfide linkages are cleaved by administration of a reducing agent, such as glutathione or ascorbate, or by a reducing agent present *in vivo,* such as cysteine, which is present in plasma and intracellularly.

Other releasable linkages include pH sensitive bonds and bonds which are cleaved upon exposure to glucose, light or heat. By way of an example, the hydrophilic polymer chains can be attached to the liposome by a pH sensitive bond, and the plasmid-liposome complexes are targeted to a site having a pH effective to cleave the bond and release the hydrophilic chains, such as a tumor region. Exemplary pH sensitive bonds include acyloxyalkyl ether, acetal and ketal bonds. Another example is where the cleavable bond is a disulfide bond, broadly intended herein to refer to sulfur-containing bonds. Sulfur-containing bonds can be synthesized to achieve a selected degree of lability and include disulfide bonds, mixed sulfide-sulfone bonds and sulfide-sulfoxide bonds. Of the three bonds, the disulfide bond is least susceptible to thiolysis and the sulfide-sulfoxide bond is most susceptible.

Such releasable bonds are useful to tailor the rate of release of the hydrophilic polymer segment from the liposome complexes. For example, a very labile disulfide bond can be used for targeting to blood cells or endothelial cells, since these cells are readily accessible and a shorter liposome blood circulation lifetime is sufficient. At the other extreme, a long-lasting or hearty disulfide bond can be used when the target is tumor tissue or other organs where a longer liposome blood circulation lifetime is generally needed for the complexes to reach the desired target.

The releasable bond attaching the hydrophilic polymer chains to the liposome is cleaved in vivo typically as a result of change in environment, such as when the liposomes reach a specific site with a slightly lower pH, such as a region of tumor tissue, or a site with reducing conditions, such as a hypoxic tumor. Reducing conditions *in* vivo can also be effected by administration of a reducing agent, such as ascorbate, cysteine or glutathione. The cleavable bond may also be broken in response to an external stimuli, such as light or heat.

In another embodiment, the liposome complexes include an affinity moiety or targeting ligand effective to bind specifically to target cells at which the therapy is aimed. Such moieties can be attached to the surface of the liposome or to the distal ends of hydrophilic polymer chains. Exemplary moieties include antibodies, ligands for specific binding to target cell surface receptors and the like, as described, for example, in PCT application Nos. WO US94/03103, WO 98/16202 and WO 98/16201. The moiety can also be a hydrophobic segment to facilitate fusion of the complex with a target cell.

Polycationic condensing agents used to condense the dsRNA and dsRNA-encoding plasmids can be multiply charged cationic polymers, and are preferably biopolymers such as such as spermidine, spermine, polylysine, protamine, total histone, specific histone fractions such as H1, H2, H3, H4, and other polycationic polypeptides, but may also include biocompatible polymers, such as polymyxin B. It will be appreciated that these polycationic condensing agents can be used in free base or salt forms, for example, protamine sulfate and polylysine hydrobromide. In a preferred embodiment, the polycationic condensing agent is a histone, which, as referred to herein, includes total histone or specific histone fractions.

In certain embodiments, the hydrophobic segment in the polymer-lipid conjugate is a hydrophobic polypeptide sequence. Preferably, the polypeptide sequence consists of about 5-80, more preferably 10-50, most preferably 20-30, nonpolar and/or aliphatic/aromatic amino acid residues. These sequences are active in triggering fusion of certain enveloped viruses with host cells and include Parainfluenza viruses, such as Sendai, Simian Virus-5 (SV5), measles virus, Newcastle Disease Virus (NDV) and Respiratory Syncytial Virus (RSV). Other examples include human retroviruses, such as Human Immunodiffiency Virus-1 (HIV-1), the causative agent of AIDS, which infects cells by fusion of the virus envelope with the plasma membrane of the host cell. Fusion occurs at physiological (i.e., neutral) pH and is followed by injection of the viral genetic material (nucleocapsid) into the cytoplasmic compartment of the host cell.

### D. Ligand-directed formulations

In certain embodiments, the supramolecular complexes and liposomes of the subject invention can be associated with one or more ligands effective to bind to specific cell surface proteins or matrix on the target cell, thereby facilitating sequestration of the complex to target cells, and in some instances, enhancing uptake of the RNAi construct by the cell. Merely to illustrate, examples of ligands suitable for use in targeting the supramolecular complexes and liposomes of the present invention to specific cell types are listed in the Table below.

| ***Ligand*** | ***Receptor*** | ***Cell type*** |
|---|---|---|
| folate | folate receptor | epithelial carcinomas, bone marrow stem cells |
| water soluble vitamins | vitamin receptor | various cells |
| pyridoxyl phosphate | CD4 | CD4 + lymphocytes |
| apolipoproteins | LDL | liver hepatocytes, vascular endothelial cells |
| insulin | insulin receptor | |
| transferrin | transferrin receptor | endothelial cells |
| galactose | asialoglycoprotein receptor | liver hepatocytes |
| sialyl-Lewisx | E, P selectin | activated endothelial cells |
| Mac-1 | L selectin | neutrophils, leukocytes |
| VEGF | Flk-1, 2 | tumor epithelial cells |
| basic FGF | FGF receptor | tumor epithelial cells |
| EGF | EGF receptor | epithelial cells |
| VCAM-1 | a₄b₁ integrin | vascular endothelial cells |
| ICAM-1 | a_{L}b₂ integrin | vascular endothelial cells |
| PECAM-1/CD31 | aᵥb₃ integrin | vascular endothelial cells, activated platelets |
| osteopontin | aᵥb₁ integrin aᵥb₅ integrin | endothelial cells and smooth muscle cells in atherosclerotic plaques |
| RGD sequences | aᵥb₃ integrin | tumor endothelial cells, vascular smooth muscle cells |
| HIV GP 120/41 or GP120 | CD4 | CD4 + lymphocytes |

### E. Respirable RNAi constructs

Another aspect of the invention provides aerosols for the delivery of RNAi constructs to the respiratory tract. The respiratory tract includes the upper airways, including the oropharynx and larynx, followed by the lower airways, which include the trachea followed by bifurcations into the bronchi and bronchioli. The upper and lower airways are called the conductive airways. The terminal bronchioli then divide into respiratory bronchioli which then lead to the ultimate respiratory zone, the alveoli, or deep lung.

Herein, administration by inhalation may be oral and/or nasal. Examples of pharmaceutical devices for aerosol delivery include metered dose inhalers'(MDIs), dry powder inhalers (DPIs), and air-jet nebulizers. Exemplary nucleic acid delivery systems by inhalation which can be readily adapted for delivery of the subject RNAi constructs are described in, for example, U.S. patents 5,756,353; 5,858,784; and PCT applications WO98/31346; WO98/10796; WO00/27359; WO01/54664; WO02/060412. Other aerosol formulations that may be used for delivering the double-stranded RNAs are described in U.S. Patents 6,294,153; 6,344,194; 6,071,497, and PCT applications WO02/066078; WO02/053190; WO01/60420; WO00/66206. Further, methods for delivering RNAi constructs can be adapted from those used in delivering other oligonucleotides (e.g., an antisense oligonucleotide) by inhalation, such as described in Templin et al., Antisense Nucleic Acid Drug Dev, 2000; 10:359-68; Sandrasagra et al., Expert Opin Biol Ther, 2001, 1:979-83; Sandrasagra et al., Antisense Nucleic Acid Drug.Dev, 2002,12:177-81.

The human lungs can remove or rapidly degrade hydrolytically cleavable deposited aerosols over periods ranging from minutes to hours. In the upper airways, ciliated epithelia contribute to the "mucociliary excalator" by which particles are swept from the airways toward the mouth. Pavia, D., "LungMucociliary Clearance," in Aerosols and the Lung: Clinical and Experimental Aspects. Clarke, S. W. and Pavia, D., Eds., Butterworths, London, 1984. In the deep lungs, alveolar macrophages are capable of phagocytosing particles soon after their deposition. Warheit et al. Microscopy Res. Tech., 26: 412-422 (1993); and Brain, J. D., "Physiology and Pathophysiology of Pulmonary Macrophages," in The Reticuloendothelial System, S. M. Reichard and J. Filkins, Eds., Plenum, New. York., pp. 315-327, 1985. The deep lung, or alveoli, are the primary target of inhaled therapeutic aerosols for systemic delivery of RNAi constructs.

In preferred embodiments, particularly where systemic dosing with the RNAi construct is desired, the aerosoled RNAi constructs are formulated as microparticles. Microparticles having a diameter of between 0.5 and ten microns can penetrate the lungs, passing through most of the natural barriers. A diameter of less than ten microns is required to bypass the throat; a diameter of 0.5 microns or greater is required to avoid being exhaled.

In certain preferred embodiments, the subject RNAi constructs are formulated in a supramolecular complex, as described above, which have a diameter of between 0.5 and ten microns, which can be aggregated into particles having a diameter of between 0.5 and ten microns.

In other embodiments, the subject RNAi constructs are provided in liposomes or supramolecular complexes (such as described above) appropriately formulated for pulmonary delivery.

### (i). Polymers for forming Microparticles.

In addition to the supramolecular complexes described above, a number of other polymers can be used to form the microparticles. As used herein, the term "microparticles" includes microspheres (uniform spheres), microcapsules (having a core and an outer layer of polymer), and particles of irregular shape.

Polymers are preferably biodegradable within the time period over which release of the RNAi construct is desired or relatively soon thereafter, generally in the range of one year, more typically a few months, even more typically a few days to a few weeks. Biodegradation can refer to either a breakup of the microparticle, that is, dissociation of the polymers forming the microparticles and/or of the polymers themselves. This can occur as a result of change in pH from the carrier in which the particles are administered to the pH at the site of release, as in the case of the diketopiperazines, hydrolysis, as in the case of poly(hydroxy acids), by diffusion of an ion such as calcium out of the microparticle, as in the case of microparticles formed by ionic bonding of a polymer such as alginate, and by enzymatic action, as in the case of many of the polysaccharides and proteins. In some cases linear release may be most useful, although in others a pulse release or "bulk release" may provided more effective results.

Representative synthetic materials are: diketopiperazines, poly(hydroxy acids) such as poly(lactic acid), poly(glycolic acid) and copolymers thereof, polyanhydrides, polyesters such as polyorthoesters, polyamides, polycarbonates, polyalkylenes such as polyethylene, polypropylene, poly(ethylene glycol), poly(ethylene oxide), poly(ethylene terephthalate), poly vinyl compounds such as polyvinyl alcohols, polyvinyl ethers, polyvinyl esters, polyvinyl halides, polyvinylpyrrolidone, polyvinylacetate, and poly vinyl chloride, polystyrene, polysiloxanes, polymers of acrylic and methacrylic acids including poly(methyl methacrylate), poly(ethyl methacrylate), poly(butylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), poly(octadecyl acrylate), polyurethanes and co-polymers thereof, celluloses including alkyl cellulose, hydroxyalkyl celluloses, cellulose ethers, cellulose esters, nitro celluloses, methyl cellulose, ethyl cellulose, hydroxypropyl cellulose, hydroxy-propyl methyl cellulose, hydroxybutyl methyl cellulose, cellulose acetate, cellulose propionate, cellulose acetate butyrate, cellulose acetate phthalate, carboxylethyl, cellulose, cellullose triacetate, and cellulose sulphate sodium salt, poly(butic acid), poly(valeric acid), and poly(lactide-co-caprolactone).

Natural polymers include alginate and other polysaccharides including dextran and cellulose, collagen, albumin and other hydrophilic proteins, zein and other prolamines and hydrophobic proteins, copolymers and mixtures thereof. As used herein, chemical derivatives thereof refer to substitutions, additions of chemical groups, for example, alkyl, alkylene, hydroxylations, oxidations, and other modifications routinely made by those skilled in the art.

Bioadhesive polymers include bioerodible hydrogels described by H. S. Sawhney, C. P. Pathak and J .A. Hubell in Macromolecules, 1993, 26, 581-587, polyhyaluronic acids, casein, gelatin, glutin, polyanhydrides, polyacrylic acid, alginate, chitosan, and polyacrylates.

To further illustrate, the matrices can be formed of the polymers by solvent evaporation, spray drying, solvent extraction and other methods known to those skilled in the art. Methods developed for making microspheres for drug delivery are described in the literature, for example, as described by Mathiowitz and Langer, J. Controlled Release 5,13-22 (1987); Mathiowitz, et al., Reactive Polymers 6, 275-283 (1987); and Mathiowitz, et al., J. Appl. Polymer Sci. 35, 755-774 (1988). The selection of the method depends on the polymer selection, the size, external morphology, and crystallinity that is desired, as described, for example, by Mathiowitz, et al., Scanning Microscopy 4,329-340 (1990); Mathiowitz, et al., J_. Appl. Polymer Sci. 45, 125-134 (1992); and Benita, et al., J. Pharm. Sci. 73, 1721-1724 (1984).

In solvent evaporation, described for example, in Mathiowitz, et al., (1990), Benita, and U.S. Pat. No. 4,272,398 to Jaffe, the polymer is dissolved in a volatile organic solvent. The RNAi constructs, either in soluble form or dispersed as fine particles, is added to the polymer solution, and the mixture is suspended in an aqueous phase that contains a surface active agent such as poly(vinyl alcohol). The resulting emulsion is stirred until most of the organic solvent evaporates, leaving solid microspheres.

In general, the polymer can be dissolved in methylene chloride. Several different polymer concentrations can be used, for example, between 0.05 and 0.20 g/ml. After loading the solution with drug, the solution is suspended in 200 ml of vigorously stirring distilled water containing 1% (w/v) poly(vinyl alcohol) (Sigma Chemical Co., St. Louis, Mo.). After four hours of stirring, the organic solvent will have evaporated from the polymer, and the resulting microspheres will be washed with water and dried overnight in a lyophilizer.

Microspheres with different sizes (1-1000 microns, though less than 10 microns for aerosol applications) and morphologies can be obtained by this method which is useful for relatively stable polymers such as polyesters and polystyrene. However, labile polymers such as polyanhydrides may degrade due to exposure to water. For these polymers, hot melt encapsulation and solvent removal may be preferred.

In hot melt encapsulation, the polymer is first melted and then mixed with the solid particles of RNAi constructs, preferably sieved to appropriate size. The mixture is suspended in a non-miscible solvent such as silicon oil and, with continuous stirring, heated to 5°C. above the melting point of the polymer. Once the emulsion is stabilized, it is cooled until the polymer particles solidify. The resulting microspheres are washed by decantation with petroleum ether to give a free-flowing powder. Microspheres with diameters between one and 1000 microns can be obtained with this method. The external surface of spheres prepared with this technique are usually smooth and dense. This procedure is useful with water labile polymers, but is limited to use with polymers with molecular weights between 1000 and 50000.

In spray drying, the polymer is dissolved in an organic solvent such as methylene chloride (0.04 g/ml). A known amount of RNAi construct is suspended (if insoluble) or co-dissolved (if soluble) in the polymer solution. The solution or the dispersion is then spray-dried. Microspheres ranging in diameter between one and ten microns can be obtained with a morphology which depends on the selection of polymer.

Hydrogel microspheres made of gel-type polymers such as alginate or polyphosphazines or other dicarboxylic polymers can be prepared by dissolving the polymer in an aqueous solution, suspending the material to be incorporated into the mixture, and extruding the polymer mixture through a microdroplet forming device, equipped with a nitrogen gas jet. The resulting microspheres fall into a slowly stirring, ionic hardening bath, as described, for example, by Salib, et al., Pharmazeutische Industrie 40-111A, 1230 (1978). The advantage of this system is the ability to further modify the surface of the microspheres by coating them with polycationic polymers such as polylysine, after fabrication, for example, as described by Lim, et al., J. Pharm. Sci. 70, 351-354 (1981). For example, in the case of alginate, a hydrogel can be formed by ionically crosslinking the alginate with calcium ions, then crosslinking the outer surface of the microparticle with a polycation such as polylysine, after fabrication. The microsphere particle size will be controlled using various size extruders, polymer flow rates and gas flow rates.

Chitosan microspheres can be prepared by dissolving the polymer in acidic solution and crosslinking with tripolyphosphate. For example, carboxymethylcellulose (CMC) microsphere are prepared by dissolving the polymer in an acid solution and precipitating the microspheres with lead ions. Alginate/polyethyleneimine (PEI) can be prepared to reduce the amount of carboxyl groups on the alginate microcapsules.

### (ii). Pharmaceutical Compositions.

The microparticles can be suspended in any appropriate pharmaceutical carrier, such as saline, for administration to a patient. In the most preferred embodiment, the microparticles will be stored in dry or lyophilized form until immediately before administration. They can then be suspended in sufficient solution, for example an aqueous solution for administration as an aerosol, or administered as a dry powder.

### (iii). Targeted Administration.

The microparticles can be delivered to specific cells, especially phagocytic cells and organs. Phagocytic cells within the Peyer's patches appear to selectively take up microparticles administered orally. Phagocytic cells of the reticuloendothelial system also take up microparticles when administered intravenously. Endocytosis of the microparticles by macrophages in the lungs can be used to target the microparticles to the spleen, bone marrow, liver and lymph nodes.

The microparticles can also be targeted by attachment of ligands, such as those described above, which specifically or non-specifically bind to particular targets. Examples of such ligands also include antibodies and fragments including the variable regions, lectins, and hormones or other organic molecules having receptors on the surfaces of the target cells.

### (iv).Storage of the Microparticles.

In the preferred embodiment, the microparticles are stored lyophilized. The dosage is determined by the amount of encapsulated RNAi constructs, the rate of release within the pulmonary system, and the pharmacokinetics of the compound.

### (v). Delivery of Microparticles.

The microparticles can be delivered using a variety of methods, ranging from administration directly into the nasal passages so that some of the particles reach the pulmonary system, to the use of a powder instillation device, to the use of a catheter or tube reaching into the pulmonary tract. Dry powder inhalers are commercially available, although those using hydrocarbon propellants are no longer used and those relying on the intake of a breath by a patient can result in a variable dose. Examples of suitable propellants include hydrofluoroalkane propellants, such as 1,1,1,2-tetrafluoroethane (CF3CH2F) (HFA-134a) and 1,1,1,2,3,3,3-heptafluoro-n-propane (CF3CHFCF3) (HFA-227), perfluoroethane, monochloroifluoromethane, 1,1 difluoroethane, and combinations thereof.

### F. Medical Device Coatings for release of RNAi constructs

Another aspect of the invention relates to coated medical devices. For instance, in certain embodiments, the subject invention provides a medical device having a coating adhered to at least one surface, wherein the coating includes the subject polymer matrix and an RNAi construct. Such coatings can be applied to surgical implements such as screws, plates, washers, sutures, prosthesis anchors, tacks, staples, electrical leads, valves, membranes. The devices can be catheters, implantable vascular access ports, blood storage bags, blood tubing, central venous catheters, arterial catheters, vascular grafts, intraaortic balloon pumps, heart valves, cardiovascular sutures, artificial hearts, a pacemaker, ventricular assist pumps, extracorporeal devices, blood filters, hemodialysis units, hemoperfasion units, plasmapheresis units, and filters adapted for deployment in a blood vessel.

In some embodiments according to the present invention, monomers for forming a polymer are combined with an RNAi construct and are mixed to make a homogeneous dispersion of the RNAi construct in the monomer solution. The dispersion is then applied to a stent or other device according to a conventional coating process, after which the crosslinking process is initiated by a conventional initiator, such as UV light. In other embodiments according to the present invention, a polymer composition is combined with an RNAi construct to form a dispersion. The dispersion is then applied to a surface of a medical device and the polymer is cross-linked to form a solid coating. In other embodiments according to the present invention, a polymer and an RNAi construct are combined with a suitable solvent to form a dispersion, which is then applied to a stent in a conventional fashion. The solvent is then removed by a conventional process, such as heat evaporation, with the result that the polymer and RNAi construct (together forming a sustained-release drug delivery system) remain on the stent as a coating. An analogous process may be used where the RNAi construct is dissolved in the polymer composition.

In some embodiments according to the invention, the system comprises a polymer that is relatively rigid. In other embodiments, the system comprises a polymer that is soft and malleable. In still other embodiments, the system includes a polymer that has an adhesive character. Hardness, elasticity, adhesive, and other characteristics of the polymer are widely variable, depending upon the particular final physical form of the system, as discussed in more detail below.

Embodiments of the system according to the present invention take many different forms. In some embodiments, the system consists of the RNAi construct suspended or dispersed in the polymer. In certain other embodiments, the system consists of an RNAi construct and a semi solid or gel polymer, which is adapted to be injected via a syringe into a body. In other embodiments according to the present invention, the system consists of an RNAi construct and a soft flexible polymer, which is adapted to be inserted or implanted into a body by a suitable surgical method. In still further embodiments according to the present invention, the system consists of a hard, solid polymer, which is adapted to be inserted or implanted into a body by a suitable surgical method. In further embodiments, the system comprises a polymer having the RNA construct suspended or dispersed therein, wherein the RNAi construct and polymer mixture forms a coating on a surgical implement, such as a screw, stent, pacemaker, etc. In particular embodiments according to the present invention, the device consists of a hard, solid polymer, which is shaped in the form of a surgical implement such as a surgical screw, plate, stent, etc., or some part thereof. In other embodiments according to the present invention, the system includes a polymer that is in the form of a suture having the RNAi construct dispersed or suspended therein.

In some embodiments according to the present invention, provided is a medical device comprising a substrate having a surface, such as an exterior surfaces and a coating on the exterior surface. The coating comprises a polymer and an RNAi construct dispersed in the polymer, wherein the polymer is permeable to the RNAi construct or biodegrades to release the RNAi construct. In certain embodiments according to the present invention, the device comprises an RNAi construct suspended or dispersed in a suitable polymer, wherein the RNAi construct and polymer are coated onto an entire substrate, e.g., a surgical implement. Such coating may be accomplished by spray coating or dip coating.

In other embodiments according to the present invention, the device comprises an RNAi construct and polymer suspension or dispersion, wherein the polymer is rigid, and forms a constituent part of a device to be inserted or implanted into a body. For instance, in particular embodiments according to the present invention, the device is a surgical screw, stent, pacemaker, etc. coated with the RNAi construct suspended or dispersed in the polymer. In other particular embodiments according to the present invention, the polymer in which the RNAi construct is suspended forms a tip or a head, or part thereof, of a surgical screw. In other embodiments according to the present invention, the polymer in which RNAi construct is suspended or dispersed is coated onto a surgical implement such as surgical tubing (such as colostomy, peritoneal lavage, catheter, and intravenous tubing). In still further embodiments according to the present invention, the device is an intravenous needle having the polymer and RNAi construct coated thereon.

As discussed above, the coating according to the present invention comprises a polymer that is bioerodible or non bioerodible. The choice of bioerodible versus non-bioerodible polymer is made based upon the intended end use of the system or device. In some embodiments according to the present invention, the polymer is advantageously bioerodible. For instance, where the system is a coating on a surgically implantable device, such as a screw, stent, pacemaker, etc., the polymer is advantageously bioerodible. Other embodiments according to the present invention in which the polymer is advantageously bioerodible include devices that are implantable, inhalable, or injectable suspensions or dispersions of RNAi construct in a polymer, wherein the further elements (such as screws or anchors) are not utilized.

In some embodiments according to the present invention wherein the polymer is poorly permeable and bioerodible, the rate of bioerosion of the polymer is advantageously sufficiently slower than the rate of RNAi construct release so that the polymer remains in place for a substantial period of time after the RNAi construct has been released, but is eventually bioeroded and resorbed into the surrounding tissue. For example, where the device is a bioerodible suture comprising the RNAi construct suspended or dispersed in a bioerodible polymer, the rate of bioerosion of the polymer is advantageously slow enough that the RNAi construct is released in a linear manner over a period of about three to about 14 days, but the sutures persist for a period of about three weeks to about six months. Similar devices according to the present invention include surgical staples comprising an RNAi construct suspended or dispersed in a bioerodible polymer.

In other embodiments according to the present invention, the rate of bioerosion of the polymer is advantageously on the same order as the rate of RNAi construct release. For instance, where the system comprises an RNAi construct suspended or dispersed in a polymer that is coated onto a surgical implement, such as an orthopedic screw, a stent, a pacemaker, or a non-bioerodible suture, the polymer advantageously bioerodes at such a rate that the surface area of the RNAi construct that is directly exposed to the surrounding body tissue remains substantially constant over time.

In other embodiments according to the present invention, the polymer vehicle is permeable to water in the surrounding tissue, e.g. in blood plasma. In such cases, water solution may permeate the polymer, thereby contacting the RNAi, construct. The rate of dissolution may be governed by a complex set of variables, such as the polymer's permeability, the solubility of the RNAi construct, the pH, ionic strength, and protein composition, etc. of the physiologic fluid

In some embodiments according to the present invention, the polymer is non-bioerodible. Non bioerodible polymers are especially useful where the system includes a polymer intended to be coated onto, or form a constituent part, of a surgical implement that is adapted to be permanently, or semi permanently, inserted or implanted into a body. Exemplary devices in which the polymer advantageously forms a permanent coating on a surgical implement include an orthopedic screw, a stent, a prosthetic joint, an artificial valve, a permanent suture, a pacemaker, etc.

There are a multiplicity of different stents that may be utilized following percutaneous transluminal coronary angioplasty. Although any number of stents may be utilized in accordance with the present invention, for simplicity, a limited number of stents will be described in exemplary embodiments of the present invention. The skilled artisan will recognize that any number of stents may be utilized in connection with the present invention. In addition, as stated above, other medical devices may be utilized.

A stent is commonly used as a tubular structure left inside the lumen of a duct to relieve an obstruction. Commonly, stents are inserted into the lumen in a non-expanded form and are then expanded autonomously, or with the aid of a second device in situ. A typical method of expansion occurs through the use of a catheter-mounted angioplasty balloon which is inflated within the stenosed vessel or body passageway in order to shear and disrupt the obstructions associated with the wall components of the vessel and to obtain an enlarged lumen.

The stents of the present invention may be fabricated utilizing any number of methods. For example, the stent may be fabricated from a hollow or formed stainless steel tube that may be machined using lasers, electric discharge milling, chemical etching or other means. The stent is inserted into the body and placed at the desired site in an unexpanded form. In one exemplary embodiment, expansion may be effected in a blood vessel by a balloon catheter, where the final diameter of the stent is a function of the diameter of the balloon catheter used.

It should be appreciated that a stent in accordance with the present invention may be embodied in a shape-memory material, including, for example, an appropriate alloy of nickel and titanium or stainless steel.

Structures formed from stainless steel may be made self-expanding by configuring the stainless steel in a predetermined manner, for example, by twisting it into a braided configuration. In this embodiment after the stent has been formed it may be compressed so as to occupy a space sufficiently small as to permit its insertion in a blood vessel or other tissue by insertion means, wherein the insertion means include a suitable catheter, or flexible rod.

On emerging from the catheter, the stent may be configured to expand into the desired configuration where the expansion is automatic or triggered by a change in pressure, temperature or electrical stimulation.

Regardless of the design of the stent, it is preferable to have the RNAi construct applied with enough specificity and a sufficient concentration to provide an effective dosage in the lesion area. In this regard, the "reservoir size" in the coating is preferably sized to adequately apply the RNAi construct at the desired location and in the desired amount.

In an alternate exemplary embodiment, the entire inner and outer surface of the stent may be coated with the RNAi construct in therapeutic dosage amounts. It is, however, important to note that the coating techniques may vary depending on the RNAi construct. Also, the coating techniques may vary depending on the material comprising the stent or other intraluminal medical device.

The intraluminal medical device comprises the sustained release drug delivery coating. The RNAi construct coating may be applied to the stent via a conventional coating process, such as impregnating coating, spray coating and dip coating.

In one embodiment, an intraluminal medical device comprises an elongate radially expandable tubular stent having an interior luminal surface and an opposite exterior surface extending along a longitudinal stent axis. The stent may include a permanent implantable stent, an implantable grafted stent, or a temporary stent, wherein the temporary stent is defined as a stent that is expandable inside a vessel and is thereafter retractable from the vessel. The stent configuration may comprise a coil stent, a memory coil stent, a Nitinol stent, a mesh stent, a scaffold stent, a sleeve stent, a permeable stent, a stent having a temperature sensor, a porous stent, and the like. The stent may be deployed according to conventional methodology, such as by an inflatable balloon catheter, by a self-deployment mechanism (after release from a catheter), or by other appropriate means. The elongate radially expandable tubular sent may be a grafted stent, wherein the grafted stent is a composite device having a stent inside or outside of a graft. The graft may be a vascular graft, such as an ePTFE graft, a biological graft, or a woven graft.

The RNAi construct may be incorporated onto or affixed to the stent in a number of ways. In the exemplary embodiment, the RNAi construct is directly incorporated into a polymeric matrix and sprayed onto the outer surface of the stent. The RNAi construct elutes from the polymeric matrix over time and enters the surrounding tissue. The RNAi construct preferably remains on the stent for at least three days up to approximately six months, and more preferably between seven and thirty days.

In certain embodiments, the polymer according to the present invention comprises any biologically tolerated polymer that is permeable to the RNAi construct and while having a permeability such that it is not the principal rate determining factor in the rate of release of the RNAi construct from the polymer.

In some embodiments according to the present invention, the polymer is non-bioerodible. Examples of non-bioerodible polymers useful in the present invention include poly(ethylene-co-vinyl acetate) (EVA), polyvinylalcohol and polyurethanes, such as polycarbonate-based polyurethanes. In other embodiments of the present invention, the polymer is bioerodible. Examples of bioerodible polymers useful in the present invention include polyanhydride, polylactic acid, polyglycolic acid, polyorthoester, polyalkylcyanoacrylate or derivatives and copolymers thereof. The skilled artisan will recognize that the choice of bioerodibility or non-bioerodibility of the polymer depends upon the final physical form of the system, as described in greater detail below. Other exemplary polymers include polysilicone and polymers derived from hyaluronic acid. The skilled artisan will understand that the polymer according to the present invention is prepared under conditions suitable to impart permeability such that it is not the principal rate determining factor in the release of the RNAi construct from the polymer.

Moreover, suitable polymers include naturally occurring (collagen, hyaluronic acid, etc.) or synthetic materials that are biologically compatible with bodily fluids and mammalian tissues, and essentially insoluble in bodily fluids with which the polymer will come in contact. In addition, the suitable polymers essentially prevent interaction between the RNAi construct dispersed/suspended in the polymer and proteinaceous components in the bodily fluid. The use of rapidly dissolving polymers or polymers highly soluble in bodily fluid or, which permit interaction between the RNAi construct and proteinaceous components are to be avoided in certain instances since dissolution of the polymer or interaction with proteinaceous components would affect the constancy of drug release.

Other suitable polymers include polypropylene, polyester, polyethylene vinyl acetate (PVA or EVA), polyethylene oxide (PEO), polypropylene oxide, polycarboxylic acids, polyalkylacrylates, cellulose ethers, silicone, poly(d1-lactide-co glycolide), various Eudragrits (for example, NE30D, RS PO and RL PO), polyalkyl-alkyacrylate copolymers, polyester-polyurethane block copolymers, polyether-polyurethane block copolymers, polydioxanone, poly-(*β-*hydroxybutyrate), polylactic acid (PLA), polycaprolactone, polyglycolic acid, and PEO-PLA copolymers.

The coating of the present invention may be formed by mixing one or more suitable monomers and a suitable RNAi construct, then polymerizing the monomer to form the polymer system. In this way, the RNAi construct is dissolved or dispersed in the polymer. In other embodiments, the RNAi construct is mixed into a liquid polymer or polymer dispersion and then the polymer is further processed to form the inventive coating. Suitable further processing may include crosslinking with suitable crosslinking RNAi constructs, further polymerization of the liquid polymer or polymer dispersion, copolymerization with a suitable monomer, block copolymerization with suitable polymer blocks, etc. The further processing traps the RNAi construct in the polymer so that the RNAi construct is suspended or dispersed in the polymer vehicle.

Any number of non-erodible polymers may be utilized in conjunction with the RNAi construct. Film-forming polymers that can be used for coatings in this application can be absorbable or non-absorbable and must be biocompatible to minimize irritation to the vessel wall. The polymer may be either biostable or bioabsorbable depending on the desired rate of release or the desired degree of polymer stability, but a bioabsorbable polymer may be preferred since, unlike biostable polymer, it will not be present long after implantation to cause any adverse, chronic local response. Furthermore, bioabsorbable polymers do not present the risk that over extended periods of time there could be an adhesion loss between the stent and coating caused by the stresses of the biological environment that could dislodge the coating and introduce further problems even after the stent is encapsulated in tissue.

Suitable film-forming bioabsorbable polymers that could be used include polymers selected from the group consisting of aliphatic polyesters, poly(amino acids), copoly(ether-esters)_{;} polyalkylenes oxalates, polyamides, poly(iminocarbonates), polyorthoesters, polyoxaesters, polyamidoesters, polyoxaesters containing amido groups, poly(anhydrides), polyphosphazenes, biomolecules and blends thereof. For the purpose of this invention aliphatic polyesters include homopolymers and copolymers of lactide (which includes lactic acid d-,1- and meso lactide), ε-caprolactone, glycolide (including glycolic acid), hydroxybutyrate, hydroxyvalerate, para-dioxanone, trimethylene carbonate (and its alkyl derivatives), 1,4-dioxepan-2-one, 1,5-dioxepan-2-one, 6,6-dimethyl-1,4-dioxan-2₋one and polymer blends thereof. Poly(iminocarbonate) for the purpose of this invention include as described by Kemnitzer and Kohn, in the Handbook of Biodegradable Polymers, edited by Domb, Kost and Wisemen, Hardwood Academic Press, 1997, pages 251-272. Copoly(ether-esters) for the purpose of this invention include those copolyester-ethers described in Journal of Biomaterials Research, Vol. 22, pages 993-1009, 1988 by Cohn and Younes and Cohn, Polymer Preprints (ACS Division of Polymer Chemistry) Vol. 30(1), page 498, 1989 (e.g. PEO/PLA). Polyalkylene oxalates for the purpose of this invention include U.S. Pat. Nos. 4,208,511; 4,141,087; 4,130,639; 4,140,678; 4,105,034; and 4,205,399 (incorporated by reference herein). Polyphosphazenes, co-, ter- and higher order mixed monomer based polymers made from L-lactide, D,L-lactide, lactic acid, glycolide, glycolic acid, para-dioxanone, trimethylene carbonate and ε-caprolactone such as are described by Allcock in The Encyclopedia of Polymer Science, Vol. 13, pages 31-41, Wiley Intersciences, John Wiley & Sons, 1988 and by Vandorpe, Schacht, Dejardin and Lemmouchi in the Handbook of Biodegradable Polymers, edited by Domb, Kost and Wisemen, Hardwood Academic Press, 1997, pages 161-182 (which are hereby incorporated by reference herein). Polyanhydrides from diacids of the form HOOC—C₆H₄-O-(CH₂)ₘ-O-C₆H₄-COOH where m is an integer in the range of from 2 to 8 and copolymers thereof with aliphatic alpha-omega diacids of up to 12 carbons. Polyoxaesters polyoxaamides and polyoxaesters containing amines and/or amido groups are described in one or more of the following U.S. Pat. Nos. 5,464,929; 5,595,751; 5,597,579; 5,607,687; 5,618,552; 5,620,698; 5,645,850; 5,648,088; 5,698,213 and 5,700,583; (which are incorporated herein by reference). Polyorthoesters such as those described by Heller in Handbook of Biodegradable Polymers, edited by Domb, Kost and Wisemen, Hardwood Academic Press, 1997, pages 99-118 (hereby incorporated herein by reference). Film-forming polymeric biomolecules for the purpose of this invention include naturally occurring materials that may be enzymatically degraded in the human body or are hydrolytically unstable in the human body such as fibrin, fibrinogen, collagen, elastin, and absorbable biocompatable polysaccharides such as chitosan, starch, fatty acids (and esters thereof), glucoso-glycans and hyaluronic acid.

Suitable film-forming biostable polymers with relatively low chronic tissue response, such as polyurethanes, silicones, poly(meth)acrylates, polyesters, polyalkyl oxides (polyethylene oxide), polyvinyl alcohols, polyethylene glycols and polyvinyl pyrrolidone, as well as, hydrogels such as those formed from crosslinked polyvinyl pyrrolidinone and polyesters could also be used. Other polymers could also be used if they can be dissolved, cured or polymerized on the stent. These include polyolefins, polyisobutylene and ethylene-alphaolefin copolymers; acrylic polymers (including methacrylate) and copolymers, vinyl halide polymers and copolymers, such as polyvinyl chloride; polyvinyl ethers, such as polyvinyl methyl ether; polyvinylidene halides such as polyvinylidene fluoride and polyvinylidene chloride; polyacrylonitrile, polyvinyl ketones; polyvinyl aromatics such as polystyrene; polyvinyl esters such as polyvinyl acetate; copolymers of vinyl monomers with each other and olefins, such as etheylene-methyl methacrylate copolymers, acrylonitrile-styrene copolymers, ABS resins and ethylene-vinyl acetate copolymers; polyamides,such as Nylon 66 and polycaprolactam; alkyd resins; polycarbonates; polyoxymethylenes; polyimides; polyethers; epoxy resins, polyurethanes; rayon; rayon-triacetate, cellulose, cellulose acetate, cellulose acetate butyrate; cellophane; cellulose nitrate; cellulose propionate; cellulose ethers (i.e. carboxymethyl cellulose and hydoxyalkyl celluloses); and combinations thereof. Polyamides for the purpose of this application would also include polyamides of the form -NH-(CH₂)ₙ-CO- and NH-(CH₂)ₓ-NH-CO-(CH₂)_{y}CO, wherein n is preferably an integer in from 6 to 13; x is an integer in the range of form 6 to 12; and y is an integer in the range of from 4 to 16. The list provided above is illustrative but not limiting.

The polymers used for coatings can be film-forming polymers that have molecular weight high enough as to not be waxy or tacky. The polymers also should adhere to the stent and should not be so readily deformable after deposition on the stent as to be able to be displaced by hemodynamic stresses. The polymers molecular weight be high enough to provide sufficient toughness so that the polymers will not to be rubbed off during handling or deployment of the stent and must not crack during expansion of the stent. In certain embodiments, the polymer has a melting temperature above 40°C, preferably above about 45°C, more preferably above 50°C and most preferably above 55°C.

Coating may be formulated by mixing one or more of the therapeutic RNAi constructs with the coating polymers in a coating mixture. The RNAi construct may be present as a liquid, a finely divided solid, or any other appropriate physical form. Optionally, the mixture may include one or more additives, e.g., nontoxic auxiliary substances such as diluents, carriers, excipients, stabilizers or the like. Other suitable additives may be formulated with the polymer and RNAi construct. For example, hydrophilic polymers selected from the previously described lists of biocompatible film forming polymers may be added to a biocompatible hydrophobic coating to modify the release profile (or a hydrophobic polymer may be added to a hydrophilic coating to modify the release profile). One example would be adding a hydrophilic polymer selected from the group consisting of polyethylene oxide, polyvinyl pyrrolidone, polyethylene glycol, carboxylmethyl cellulose, hydroxymethyl cellulose and combination thereof to an aliphatic polyester coating to modify the release profile. Appropriate relative amounts can be determined by monitoring the in *vitro* and/or *in vivo* release profiles for the therapeutic RNAi constructs.

The thickness of the coating can determine the rate at which the RNAi construct elutes from the matrix. Essentially, the RNAi construct elutes from the matrix by diffusion through the polymer matrix. Polymers are permeable, thereby allowing solids, liquids and gases to escape therefrom. The total thickness of the polymeric matrix is in the range from about one micron to about twenty microns or greater. It is important to note that primer layers and metal surface treatments may be utilized before the polymeric matrix is affixed to the medical device. For example, acid cleaning, alkaline (base) cleaning, salinization and parylene deposition may be used as part of the overall process described.

To further illustrate, a poly(ethylene-co-vinylacetate), polybutylmethacrylate and RNAi construct solution may be incorporated into or onto the stent in a number of ways. For example, the solution may be sprayed onto the stent or the stent may be dipped into the solution. Other methods include spin coating and RF plasma polymerization. In one exemplary embodiment, the solution is sprayed onto the stent and then allowed to dry. In another exemplary embodiment, the solution may be electrically charged to one polarity and the stent electrically changed to the opposite polarity. In this manner, the solution and stent will be attracted to one another. In using this type of spraying process, waste may be reduced and more precise control over the thickness of the coat may be achieved.

In another exemplary embodiment, the RNAi construct may be incorporated into a film-forming polyfluoro copolymer comprising an amount of a first moiety selected from the group consisting of polymerized vinylidenefluoride and polymerized tetrafluoroethylene, and an amount of a second moiety other than the first moiety and which is copolymerized with the first moiety, thereby producing the polyfluoro copolymer, the second moiety being capable of providing toughness or elastomeric properties to the polyfluoro copolymer, wherein the relative amounts of the first moiety and the second moiety are effective to provide the coating and film produced therefrom with properties effective for use in treating implantable medical devices.

In one embodiment according to the present invention, the exterior surface of the expandable tubular stent of the intraluminal medical device of the present invention comprises a coating according to the present invention. The exterior surface of a stent having a coating is the tissue-contacting surface and is biocompatible. The "sustained release RNAi construct delivery system coated surface" is synonymous with "coated surface", which surface is coated, covered or impregnated with a sustained release RNAi construct delivery system according to the present invention.

In an alternate embodiment, the interior luminal surface or entire surface (i.e. both interior and exterior surfaces) of the elongate radially expandable tubular stent of the intraluminal medical device of the present invention has the coated surface. The interior luminal surface having the inventive sustained release RNAi construct delivery system coating is also the fluid contacting surface, and is biocompatible and blood compatible.

### V. Exemplary Uses

In one aspect, the subject method is used to inhibit, or at least reduce, unwanted growth of cells *in vivo,* and particularly the growth of transformed cells. In certain embodiments, the subject method utilizes RNAi to selectively inhibit the expression of genes encoding proliferation-regulating proteins. For instance, the subject method can be used to inhibit expression of a gene product that is essential to mitosis in the target cell, and/or which is essential to preventing apoptosis of the target cell. The RNAi constructs of the present invention can be designed to correspond to the coding sequence or other portions of mRNAs encoding the targeted proliferation-regulating protein. When treated with the RNAi construct, the loss-of-expression phenotype which results in the target cell causes the cell to become quiescent or to undergo apoptosis.

In certain embodiments, the subject RNAi constructs are selected to inhibit expression of gene products which stimulate cell growth and mitosis. On class of genes which can be targeted by the method of the present invention are those known as oncogenes. As used herein, the term "oncogene" refers to a gene which stimulates cell growth and, when its level of expression in the cell is reduced, the rate of cell growth is reduced or the cell becomes quiescent. In the context of the present invention, oncogenes include intracellular proteins, as well as extracellular growth factors which may stimulate cell proliferation through autocrine or paracrine function. Examples of human oncogenes against which RNAi constructs can designed include c-myc, c-myb, mdm2, PKA-I (protein kinase A type I), Abl-1, Bcl2, Ras, c-Raf kinase, CDC25 phosphatases, cyclins, cyclin dependent kinases (cdks), telomerase, PDGF/sis, erb-B, fos, jun, mos, and src, to name but a few. In the context of the present invention, oncogenes also include a fusion gene resulted from chromosomal translocation, for example, the Bcr/Ab1 fusion oncogene.

In certain preferred embodiments, the subject RNAi constructs are selected by their ability to inhibit expression of a gene(s) essential for proliferation of a transformed cell, and particularly of a tumor cell. Such RNAi constructs can be used as part of the treatment or prophylaxis for neoplastic, anaplastic and/or hyperplastic cell growth *in vivo,* including as part of a treatment of a tumor. The c-myc protein is deregulated in many forms of cancer, resulting in increased expression. Reduction of c-myc RNA levels *in vitro* results in induction of apoptosis. An siRNA complementary to c-myc can therefore be potentially be used as therapeutic for anticancer treatment. Preferably, the subject RNAi constructs can be used in the therapeutic treatment of chronic lymphatic leukemia. Chronic lymphatic leukemia is often caused by a translocation of chromosomes 9 and 12 resulting in a Bcr/Ab1 fusion product. The resulting fusion protein acts as an oncogene; therefore, specific elimination of Bcr/Ab1 fusion mRNA may result in cell death in the leukemia cells. Indeed, transfection of siRNA molecules specific for the Bcr/Ab1 fusion mRNA into cultured leukemic cells, not only reduced the fusion mRNA and corresponding oncoprotein, but also induced apoptosis of these cells (see, for example, Wilda et al., Oncogene, 2002, 21:5716-5724).

In other embodiments, the subject RNAi constructs are selected by their ability to inhibit expression of a gene(s) essential for activation of lymphocytes, e.g., proliferation of B-cells or T-cells, and particularly of antigen-mediated activation of lymphocytes. Such RNAi constructs can be used as immunosuppressant agents, e.g., as part of the treatment or prophylaxis for immune-mediated inflammatory disorders.

In certain embodiments, the methods described herein can be employed for the treatment of autoimmune disorders. For example, the subject RNAi constructs are selected for their ability to inhibit expression of a gene(s) which encode or regulate the expression of cytokines. Accordingly, constructs that cause inhibited or decreased expression of cytokines such as THFα, IL-1α, IL-6 or IL-12, or a combination thereof, can be used as part of a treatment or prophylaxis for rheumatoid arthritis. Similarly, constructs that cause inhibited or decreased expression of cytokines involved in inflammation can be used in the treatment or prophylaxis of inflammation and inflammation-related diseases, such as multiple sclerosis.

In other embodiments, the subject RNAi constructs are selected for their ability to inhibit expression of a gene(s) implicated in the onset or progression of diabetes. For example, experimental diabetes mellitus was found to be related to an increase in expression of p21WAF1/CIP1 (p21), and TGF-beta 1 has been implicated in glomerular hypertrophy (see, for example, Al-Douahji, et al. Kidney Int. 56:1691-1699). Accordingly, constructs that cause inhibited or decreased expression of these proteins can be used in the treatment or prophylaxis of diabetes.

In other embodiments, the subject RNAi constructs are selected for their ability to inhibit expression of ICAM-1 (intracellular adhesion molecule). An antisense nucleic acid that inhibits expression of ICAM-1 is being developed by Isis pharmaceutics for psoriasis. Additionally, an antisense nucleic acid against the ICAM-1 gene is suggested for preventing acute renal failure and reperfusion injury and for prolonging renal isograft survival (see, for example, Haller et al. (1996) Kidney Int. 50:473-80; Dragun et al. (1998) Kidney Int. 54:590-602; Dragun et al. (1998) Kidney Int. 54:2113-22). Accordingly, the present invention contemplates the use of RNAi constructs in the above-described diseases.

In other embodiments, the subject RNAi constructs are selected by their ability to inhibit expression of a gene(s) essential for proliferation of smooth muscle cells or other cells of endothelium of blood vessels, such as proliferating cells involved in neointima formation. In such embodiments, the subject method can be used as part of a treatment or prophylaxis for restenosis.

Merely to illustrate, RNAi constructs applied to the blood vessel endothelial cells after angioplasty can reduce proliferation of these cells after the procedure. Merely to illustrate, a specific example is an siRNA complementary to c-myc (an oncogene). Down-regulation of c-myc inhibits cell growth. Therefore, siRNA can be prepared by synthesizing the following oligonucleotides:
5'-UCCCGCGACGAUGCCCCUCATT-3'
3'-TTAGGGCGCUGCUACGGGGAGU-5'

All bases are ribonucleic acids except the thymidines shown in bold, which are deoxyribose nucleic acids (for more stability). Double-stranded RNA can be prepared by mixing the oligonucleotides at equimolar concentrations in 10 mM Tris-Cl (pH 7.0) and 20 mM NaCl , heating to 95°C, and then slowly cooling to 37 °C. The resulting siRNAs can then be purified by agarose gel electrophoresis and delivered to cells either free or complexed to a delivery system such as a cyclodextrin-based polymer. For *in vitro* experiments, the effect of the siRNA can be monitored by growth curve analysis, RT-PCR or western blot analysis for the c-myc protein.

It is demonstrated that antisense oligodeoxynucleotides directed against the c-myc gene inhibit restenosis when given by local delivery immediately after coronary stent implantation (see, for example, Kutryk et al. (2002) J Am Coll Cardiol. 39:281-287; Kipshidze et al. (2002) J Am Coll Cardiol. 39:1686-1691). Therefore, the present invention contemplates delivering an RNAi construct against the c-Myc gene (i.e., c-Myc RNAi construct) to the stent implantation site with an infiltrator delivery system (Interventional Technologies, San Diego, California). Preferably, the c-Myc RNAi construct is directly coated on stents for inhibiting restenosis. Similarly, the c-Myc RNAi construct can be delivered locally for inhibiting myointimal hyperplasia after percutaneous transluminal coronary angioplasty (PTCA) and exemplary methods of such local delivery can be found, for example, Kipshidze et al. (2001) Catheter Cardiovasc Interv. 54:247-56. Preferably, the RNAi constructs are chemically modified with, for example, phosphorothioates or phosphoramidate.

Early growth response factor-1 (i.e., Egr-1) is a transcription factor that is "activated during mechanical injury and regulates transcription of many genes involved with cell proliferation and migration. Therefore, down-regulation of this protein may also be an approach for prevention of restenosis. The siRNA directed against the Egr-1 gene can be prepared by synthesis of the following oligonucleotides:
5'-UCGUCCAGGAUGGCCGCGGTT-3'
3'-TTAGCAGGUCCUACCGGCGCC-5'

Again, all bases are ribonucleic acids except the thymidines shown in bold, which are deoxyribose nucleic acids. The siRNAs can be prepared from these oligonucleotides and introduced into cells as described herein.

### Exemplification

The invention now being generally described, it will be more readily understood by reference to the following examples, which are included merely for purposes of illustration of certain aspects and embodiments of the present invention, and are not intended to limit the invention.

### 1. Delivery of plasmid DNA encoding siRNA in vitro.

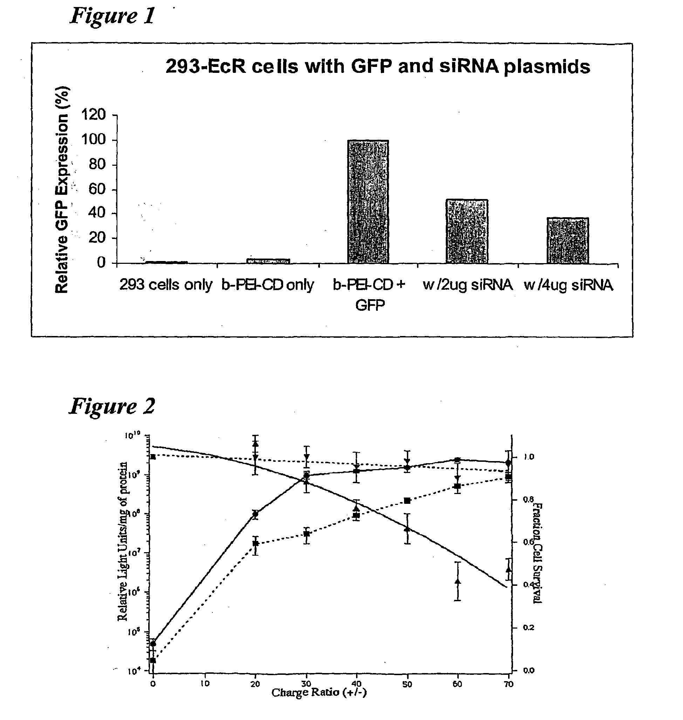

Human embryonic kidney cells (HEK 293-EcR) were seeded at 200,000 cells per well in 6-well plates. These HEK 293-EcR cells have been stably transfected with a plasmid encoding the ecdysone receptor. After 2-3 days, the cells were co-transfected with pIND-rev-GFP (a plasmid encoding inducible elements as well as green fluorescent protein) and pTZU6+1/siRNA (a plasmid encoding the sense and antisense strands of the siRNA oligonucleotides). The plasmids (see, Lee et al. (2002) Nature Biotechnology. 20:500-505) were complexed with branched PEI25k-hi-CD polymer (high degree of cyclodextrin grafting) at a ratio of 15 N/P in 0.5ml of opti-MEM. After 4 hours, the media was replaced with 2ml of complete media. At 24 hours, the cells were induced with 5µM ponasterone A to induce the GFP target gene expression. At 72 hours, the cells were removed by versene, collected, and analyzed for GFP expression by flow cytometry. As shown in Figure 1, transfection of the siRNA down-regulated GFP expression in a dose-dependent manner. An approximately 50% reduction in GFP expression was observed with 2 microgram of siRNA, while an approximately 40% reduction in GFP expression was observed with 4 microgram of siRNA. The above experiments demonstrate that RNAi constructs can be delivered successfully by β-cyclodextrin polymers in cultured cells and attenuate gene expression through an RNA inference mechanism.

### 2. DNA plasmid delivery and luciferase expression in vitro.

BHK-21 cells were plated in 24-well plates and transfected under serum-free conditions with 1 µg of the pGL3-CV plasmid (a luciferase gene-containing plasmid) complexed with the β-cyclodextrin polymers (βCDPs) at various charge ratios. Transfection efficiencies were determined by assaying for luciferase protein activity, with results reported in relative light units (RLUs) (see Figure 2). The amount of protein in cell lysates obtained 48 hours after transfection was used as a measure of cell viability. Protein levels of transfected cells were determined by Biorad's DC protein assay (Hercules, CA) and normalized with protein levels of cells transfected with naked DNA. A protein standard curve was run with various concentrations of bovine IgG (Biorad) in Cell Culture Lysis Buffer. The above experiments demonstrate that transfection efficiencies can be optimized by adjusting the charge ratio between β-cyclodextrin polymers and RNAi constructs.

### 3. DNA plasmid delivery and luciferase expression in mice.

All materials except DNA were sterilized by filtration through a 0.2 µm filter and lyophilized before use. Linear cyclodextrin polymer was prepared in 10% glucose and then added to an equal volume of the pGL3-CV plasmid (a luciferase-gene-containing plasmid) in water such that the final solutions are in 5% glucose solutions. Particles were prepared at 5+/- and at a final DNA concentration of 0.5 mg/mL. Female Balb/C mice were injected via portal vein injection with 200 µL of polymer solution containing 100 µg of luciferase DNA. Four hours after DNA administration, mice were anesthesized, injected with luciferin in the intraperitoneal cavity, and imaged for luciferase protein activity using a Xenogen camera. Luciferase expression was observed in the liver within 4 hours after plasmid administration. The above experiments demonstrate that RNAi constructs complexed with β-cyclodextrin polymers can be delivered *in vivo* (e.g., in mice).

### 4. Delivery of siRNA oligonucleotide in vitro.

Human acute leukemia K562 suspension cells (with endogenous expression of the p210 Bcr-Ab1 fusion) are seeded at 1,000,000 cells per well in 6-well plates in 0.5ml of opti-MEM. Polyplexes are formed using 2µM dsRNA (Dharmacon Research, Inc.) in 0.25ml of opti-MEM, with linear-PEI-hi-CD polymer, at a ratio of 15 N/P, also in 0.25ml of opti-MEM. The following dsRNA oligonucleotides are designed to recognize the Bcr-Ab1 fusion mRNA splice 1 target:
5'-GCAGAGUUCAAAAGCCCUUdTdT-3'
3'-dTdTCGUCUCAAGUUUUCGGGAA-5'

The 0.5ml of transfection medium is added to the 0.5ml of cells. After 4 hours, the wells are supplemented with 4ml of complete medium. At 48 hours, the cells are collected, washed, and lysed. Protein concentrations are measured and 20µg of protein are loaded and run on an SDS-PAGE gel. The proteins are transferred to a PVDF membrane, blocked with 1% BSA, and blotted with anti-Bcr antibody. p210 signal is detected by chemiluminescence (Amersham), and down-regulation is observed by a decrease in band intensity as compared with an untreated sample. The above experiments demonstrate that siRNAs formulated in supramolecular complex (e.g., β-cyclodextrin polymers) can be delivered for gene therapy of acute leukemia.

### 5. Delivery of DNAzyme in mice.

Tumor-bearing nude mice were injected with particles formulated with 1 mg of fluorescently labeled DNAzyme in 250 µL of D5W. Formulations contained CD polymer, AD-PEG, and AD-PEG-Transferrin (for tumor targeting) as described previously [Bellocq, 2002 #459]. Mice were sacrificed 24 hours after injection and tumors extracted for analysis by fluorescence stereomicroscopy. Intracellular DNAzyme localization was visualized by confocal microscopy. Penetration beyond the tumor cap and into the tumor cells is only achieved by transfenin-modified particles. The above experiments demonstrate that β-cyclodextrin polymers can be used for delivering other expression constructs (e.g., a DNAzyme) *in vivo* for gene therapy.

### 6. Delivery of long RNAs in mice.

All materials except RNA were sterilized by filtration through a 0.2 µm filter and lyophilized before use. Linear cyclodextrin polymer was prepared in 10% glucose and then added to an equal volume of luciferase RNA in water such that the final solutions are in 5% glucose solutions. Particles were prepared at 5+/- and at a final RNA concentration of 0.5 mg/mL. Female, Balb/C mice were injected via portal vein injection with 200 µL of polymer solution containing 100 µg of luciferase RNA. Four hours after RNA administration, mice were anesthesized, injected with luciferin in the intraperitoneal cavity, and imaged for luciferase protein activity using a Xenogen camera. Luciferase expression was observed in the liver within 4 hours after luciferase RNA administration. The above experiments demonstrate that long RNAs complexed with β-cyclodextrin polymers can be delivered *in vivo* (e.g., in mice).

## Claims

1. A stable respiratory formulation comprising RNAi constructs formulated for pulmonary or nasal delivery of a therapeutically effective amount of said RNAi constructs to the lungs of a patient.

2. The formulation of claim 1, wherein said RNAi constructs are formulated as supramolecular complexes including a multi-dimensional polymer network.

3. The formulation of claim 2, wherein said supramolecular complexes are formed from cationic polymers.

4. The formulation of claim3, wherein said cationic polymers are selected from the group consisting of β-cyclodextrin containing polymers (βCD-polymers), poly(L)lysine (PLL), polyethylenimine (PEI), and co-polymers thereof.

5. The formulation of claim 1, wherein said RNAi constructs are formulated as microparticles having an average diameter less than 20 microns.

6. The formulation of claim 5, wherein said microparticles
a) have an average diameter of 0.5 to 10 microns,
b) are formed from biodegradable polymers,
c) are formed from one or more polymers selected from the group consisting of polysaccharides, diketopiperazines, poly(hydroxy acids), polyanhydrides, polyesters, polyamides, polycarbonates, polyalkylenes, poly vinyl compounds, polysiloxanes, polymers of acrylic and methacrylic acids, polyurethanes, celluloses, poly(valeric acid), and poly(lactide-co-caprolactone), or co-polymers thereof,
d) are formed by solvent evaporation, spray drying, solvent extraction or hot melt encapsulation, or
e) are in dry or lyophilized form.

7. The formulation of claim 2, wherein said supramolecular complexes are formed from
cyclodextrin-modified polymers,
wherein said supramolecular complexes are preferably formed from cyclodextrin-modified
poly(ethylenimine) and have a structure of the formula: 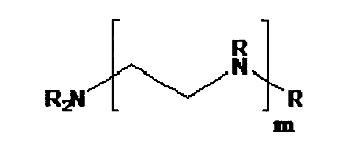 wherein
R represents, independently for each occurrence, H, lower alkyl, a cyclodextrin
moiety, or 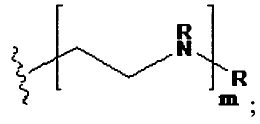 and
m, independently for each occurrence, represents an integer from 2-10,000, preferably from 10 to 5,000, or from 100 to 1,000.

8. The formulation of claim 1 or 5, wherein said RNAi constructs are formulated in liposomes.

9. The formulation of claim 1, including a propellant.

10. The formulation of claim 1, contained in a metered dose inhaler, a dry powder inhaler or an air-jet nebulizer.

11. The formulation of claim 1, wherein said RNAi construct is formulated in an amount to provide a therapeutically effective amount in one to ten meter doses.

12. The formulation of claim 1, wherein the RNAi construct includes modifications to either
phosphate-sugar backbone or the nucleoside,.
wherein the RNAi construct preferably includes a backbone modification selected from
phosphorothioates, phosphoramidate, phosphodithioates, chimeric methylphosphonate-phosphodiesters, peptide nucleic acids, and 5-propynyl-pyrimidine containing oligomers.

13. A metered dose aerosol dispenser containing an aerosol pharmaceutical composition for pulmonary or nasal delivery comprising a respirable formulation of RNAi constructs.

14. A pharmaceutical preparation comprising the formulation of claim 1 and a pharmaceutically acceptable carrier.

15. A pharmaceutical package comprising the pharmaceutical preparation of claim 14, in association with instructions (written and/or pictorial) for administering the preparation to a human patient.

16. A composition comprising one or more small interfering RNA (siRNA) formulated in a supramolecular complex formed from β-cyclodextrin containing polymers and in an amount sufficient to attenuate expression of a target gene in treated cells through an RNA interference mechanism.

17. Use of a small interfering RNA (siRNA) construct for the manufacture of a medicament for attenuating expression of a target gene of a cell *in vivo,* wherein the siRNA construct is formulated in a supramolecular complex, formed from β-cyclodextrin-containing polymers, in an amount sufficient to attenuate expression of the target gene through an RNA interference mechanism, and thereby alter the growth, survival or differentiation of treated cells.

18. The composition or use of claims 16 or 17 for use in the treatment of cells *in vivo.*

19. The composition or use of claims 16 or 17 for use in the treatment of cells *in vitro.*

20. A pharmaceutical preparation comprising the composition of claim 1 and a pharmaceutically acceptable carrier.


SEQUENCE LISTING

<110>  Davis et al.

<120>  METHODS AND COMPOSITIONS FOR THERAPEUTIC USE OF RNA INTERFERENCE

<130>  ITI-P01-001

<140>  10/288230
<141>  2002-11-04

<150>  60/336314
<151>  2001-11-02

<160>  6

<170>  PatentIn version 3.1

<210>  1
<211>  22
<212>  DNA
<213>  Artificial Sequence

<220>
<223>  Oligonucleotide for synthesis of siRNA complementary to c-myc

<400>  1
ucccgcgacg augccccuca tt                                                22

<210>  2
<211>  22
<212>  DNA
<213>  Artificial Sequence

<220>
<223>  Oligonucleotide for synthesis of siRNA complementary to c-myc

<400>  2
ttagggcgcu gcuacgggga gu                                                22

<210>  3
<211>  21
<212>  DNA
<213>  Artificial Sequence

<220>
<223>  Oligonucleotide for synthesis of siRNA directed against Egr-1
       gene

<400>  3
ucguccagga uggccgcggt t                                                 21

<210>  4
<211>  21
<212>  DNA
<213>  Artificial Sequence

<220>
<223>  Oligonucleotide for synthesis of siRNA directed against Egr-1
      gene

<400>  4
ttagcagguc cuaccggcgc c                                              21

<210>  5
<211>  21
<212>  DNA
<213>  Artificial Sequence

<220>
<223>  Oligonucleotide designed to recognize Bcr-Abl fusion mRNA splice
      1 target

<400>  5
gcagaguuca aaagcccuut t                                              21

<210>  6
<211>  21
<212>  DNA
<213>  Artificial Sequence

<220>
<223>  Oligonucleotide designed to recognize Bcr-Abl fusion mRNA splice
      1 target

<400>  6
ttcgucucaa guuuucggga a                                              21